# EUROPEAN PATENT SPECIFICATION

(11) **EP 1 418 429 B1**
(45) Date of publication and mention of the grant of the patent: **25.08.2010**
(21) Application number: 02746016.1
(22) Date of filing: 12.07.2002
(51) Int. Cl.: G01N 33/92

(54) **METHOD OF QUANTIFYING DENATURED LIPOPROTEIN**
VERFAHREN ZUR QUANTIFIZIERUNG VON DENATURIERTEM LIPOPROTEIN
PROCEDE DE QUANTIFICATION DE LIPOPROTEINES DENATUREES

(30) Priority: 12.07.2001 JP 2001212522
(43) Date of publication of application: 12.05.2004
(73) Proprietor: KYOWA MEDEX CO., LTD., Tokyo 104-0042 (JP)
(72) Inventor: KOHNO, Hiroaki, Head Office, Kyowa Medex Co., Ltd., Tokyo 104-0042 (JP); YANAGISAWA, T., T. R. L.,Kyowa Hakko Kogyo Co.,Ltd, Machida-shi, Tokyo 194-8533 (JP); MASUNARI, T., Head Office, Kyowa Medex Co., Ltd, Chuo-ku, Tokyo 104-0042 (JP)
(74) Representative: Lee, Nicholas John
(86) International application number: PCT/JP2002/007116
(87) International publication number: WO 2003/006989

(56) References cited:
- WO-A-00/02046
- JP-A- 2 216 460
- JP-A- 8 101 195
- JP-A- 8 228 774
- JP-A- 11 125 635
- JP-A- 2001 066 307
- BRILES D E ET AL: "THE EFFECTS OF SUBCLASS ON THE ABILITY OF ANTI-PHOSPHOCHOLINE ANTIBODIES TO PROTECT MICE FROM FATAL INFECTION WITH STREPTOCOCCUS PNEUMONIAE" JOURNAL OF MOLECULAR AND CELLULAR IMMUNOLOGY, SPRINGER INTERNATIONAL, NEW YORK, NY, US, vol. 1, no. 5, 1984, pages 305-309, XP009046984 ISSN: 0724-6803
- YOTHER J ET AL: "PROTECTION OF MICE FROM INFECTION WITH STREPTOCOCCUS PNEUMONIAE BY ANTI-PHOSPHOCHOLINE ANTIBODY" INFECTION AND IMMUNITY, AMERICAN SOCIETY FOR MICROBIOLOGY. WASHINGTON, US, vol. 36, no. 1, April 1982 (1982-04), pages 184-188, XP009047079 ISSN: 0019-9567

### SEQUENCE LISTING

<110> KYOWA MEDEX CO., LTD
<120> Method for determination of denatured lipoprotein and reagent therefor, and method for evaluating cardiovascular disease and reagent therefor.
<130> 11405W01
<140>
   <141>
<150> JP2001-212522
   <151> 2001-07-12
<160> 10
<170> PatentIn Ver. 2.1
<210> 1
   <211> 470
   <212> DNA
   <213> Mus musculus
<400> 1
   ctcccaatct tcacattcag aaatcagcac tcagtcctgt cactatgaag ttgtggttaa 60
   actgggtttt tcttttaaca cttttacatg gtatccagtg tgaggtgaag ctggtggaat 120
   ctggaggagg cttggtacag cctgggggtt ctctgagact ctcctgtgca acttctgggt 180
   tcaccttcag tgatttctac atggagtggg tccgccagcc tccagggaag agactggagt 240
   ggattgctgc aagtagaaac aaagctaatg attatacaac agagtacagt gcatctgtga 300
   agggtcggtt catcgtctcc agagacactt cccaaagcat cctctacctt cagatgaatg 360
   ccctgagagc tgaggacact gccatttatt actgtgcaag agattactac ggtagtagct 420
   actggtactt cgatgtctgg ggcgcaggga ccacggtcac cgtctcctca 470
<210> 2
   <211> 422
   <212> DNA
   <213> Mus musculus
<400> 2
   tgtcaaacag cagggggagc aggatggagt ttcagaccca ggtactcatg tccctgctgc 60
   tctgcatgtc tggtgcctgt gcagacattg tgatgactca gtctccaact ttccttgctg 120
   tgacagcaag taagaaggtc accattagtt gcacggccag tgagagcctt tattcaagca 180
   aacacaaggt gcactacttg gcttggtacc agaagaaacc agagcaatct cctaaactgc 240
   tgatatacgg ggcatccaac cgatacattg gggtccctga tcgcttcaca ggcagtggat 300
   ctgggacaga tttcactctg accatcagca gtgtacaggt tgaagacctc acacattatt 360
   actgtgcaca gttctacagc tatcctctca cgttcggtgc tgggaccaag ctggagctga 420
   aa                                                                422
<210> 3
   <211> 39
   <212> DNA
   <213> Artificial Sequence
<220>
   <223> Description of Artificial Sequence: Synthetic DNA
<400> 3
   gtcaccgtct cctcagcctc caccaagggc ccggatcca 39
<210> 4
   <211> 38
   <212> DNA
   <213> Artificial Sequence
<220>
   <223> Description of Artificial Sequence: Synthetic DNA
<400> 4
   ctagtggatc cgggcccttg gtggaggctg aggagacg 38
<210> 5
   <211> 42
   <212> DNA
   <213> Artificial Sequence
<220>
   <223> Description of Artificial Sequence: Synthetic DNA
<400> 5
   tacagctatc ctctcacgtt cggtgctggg accaagctgg ag 42
<210> 6
   <211> 54
   <212> DNA
   <213> Artificial Sequence
<220>
   <223> Description of Artificial Sequence: Synthetic DNA
<400> 6
   gatcccgtac gtttcagctc cagcttggtc ccagcaccga acgtgagagg atag 54
<210> 7
   <211> 27
   <212> DNA
   <213> Artificial Sequence
<220>
   <223> Description of Artificial Sequence: Synthetic DNA
<400> 7
   ggttcacctt cagtgatttc tacatgg 27
<210> 8
   <211> 23
   <212> DNA
   <213> Artificial Sequence
<220>
   <223> Description of Artificial Sequence: Synthetic DNA
<400> 8
   ccagacatcg aagtaccagt agc 23
<210> 9
   <211> 26
   <212> DNA
   <213> Artificial Sequence
<220>
   <223> Description of Artificial Sequence: Synthetic DNA
<400> 9
   gagcctttat tcaagcaaac acaagg 26
<210> 10
   <211> 26
   <212> DNA
   <213> Artificial Sequence
<220>
   <223> Description of Artificial Sequence: Synthetic DNA
<400> 10
   gaggatagct gtagaactgt gcacag 26

## Description

### Technical Field

The present invention relates to a method for the quantitative determination of denatured lipoprotein in a biological sample which comprises determining the denatured lipoprotein in the biological sample by using an antibody against phosphocholine, a method for the quantitative determination of a denatured lipoprotein in a biological sample which comprises determining the denatured lipoprotein in the biological sample by using an antibody against phosphocholine and an antibody against lipoprotein, a method for the detection of a cardiovascular disease which comprises determining a denatured lipoprotein in a biological sample by using an antibody against phosphocholine and detecting the cardiovascular disease based on the result of the determination and a method for the detection of a cardiovascular disease which comprises determining a denatured lipoprotein in a biological sample by using an antibody against phosphocholine and an antibody against lipoprotein and detecting the cardiovascular disease based on the result of the determination. Also described herein is a reagent for the determination of a denatured lipoprotein containing an antibody against phosphocholine, and a reagent for the determination of a denatured lipoprotein containing an antibody against phosphocholine and an antibody against lipoprotein.

### Background Art

The arteriosclerosis occurs mostly in muscular type artery such as aorta, coronary artery, cerebral artery and carotid artery, and is a main cause for angina pectoris, myocardial infarction, and cerebral infarction. Though the increased serum cholesterol, platelet aggregation, endothelial injury, etc. have been suggested as causes therefor, their causes have been revealed scarcely.

It has been suggested that serum lipid is closely related to various circulatory diseases including diseases of coronary system such as myocardial infarction and angina pectoris, diseases of cerebral artery system such as cerebral infarction and cerebral vascular dementia, diseases of renal artery such as nephropathy and diabetic nephropathy, and diseases of peripheral artery such as peripheral arterial occlusion. It has been considered that the determination of the serum lipid is very important for the diagnosis of these diseases, the elucidation of the pathosis, and the detection of therapeutic effects.

The recent researches devoted to the comparison of absolute amounts of serum lipid between a group of patients of the diseases mentioned above and a group of healthy persons have produced a report that no large difference exists between the two groups and that rather the amounts of lipoprotein subjected to denaturation (hereinafter referred to as "denatured lipoprotein") present in serum were clearly different between the two groups [as disclosed in Circulation, 94, Suppl. I, 1288 (1996), for example]. The relationship between the oxidized lipoprotein which is one of the denatured lipoproteins and the development of lesion of atherosclerosis has been indicated by Steinberg et al. [as disclosed in N. Engl. J. Med., 320, 915 (1989), for example].

Further, the presence of such receptors for the denatured lipoprotein as a scavenger receptor has been revealed. The hypothesis that the denatured lipoprotein, on being entrapped via such a receptor into a cell, is transformed into a foam cell and induces the initiation of the formation of atheroma and the hypothesis that the denatured lipoprotein, on injuring an endothelial cell, induces the adhesion and aggregation of platelets, assembly of leucocytes, and infiltration of blood cells into a vessel and consequently triggers inducing smooth muscle cells to migrate and proliferate have been advanced.

As regards the accumulation of a denatured lipoprotein at lesion, Haberland et al. have reported in 1988 that the lesion of arteriosclerosis is stained by an antibody against a low-density lipoprotein (LDL) modified with malondialdehyde [anti-malondialdehyde (MDA)-LDL antibody] [Science, 241, 215 (1988)] and Yla Herttuala et al. have reported in 1989 that an Apo B extracted from a lesion is detected by an immunoblotting method using an anti-MDA-ApoB antibody [J. Clin. Invest., 84, 1086 (1989)]. Since the anti-MDA-ApoB antibody mentioned above, an antibody obtained by using as an antigen a lipoprotein artificially modified by oxidation with malondialdehyde, shows a cross reaction with not only an oxidized product but also other denatured proteins such as oxidized albumin, however, it is unknown whether the denatured lipoprotein is indeed determined as asserted in the report.

As denatured lipoproteins, acetylated lipoprotein, glycosylated lipoprotein, malonaldehyde-modified lipoprotein, 4-hydroxy-2-nonenal (HNE)-modified lipoprotein, and oxidized lipoprotein have been known. The *in vivo* entity of a denatured lipoprotein, however, remains yet to be elucidated.

It has been known that a lipoprotein, on inducing aggregation, is made to change its nature and tend to be incorporated into macrophage [J. Biol. Chem., 272, 31700, (1997)]. This change is thought to be one type of denaturation of a lipoprotein. This denaturation has been known to be induced by oxidation, conversion into a thiol, or a certain type of enzymatic treatment [Arterioscier, Thromb., 11, 1209 (1991), Arch. Biachem. Biophys., 310, 489 (1994), Biochim. Biophys. Acta., 1215, 79 (1994), Nutr. Metab. Cardiovasc. Dis., 4, 70 (1994), Proc. Nati. Acad. Sci. U.S.A., 86, 2713 (1989), Arterioscier. Thromb., 11, 1643 (1991), J. Biol. Chem., 268, 20419 (1993), and Circ. Res., 71, 118 (1992)]. Such a denaturation is induced by such a physical treatment as Vortex Treatment [Arteriosclerosis, 8, 348 (1988)]. The lipoprotein which is subjected to such denaturation is easily incorporated into macrophage. Thus, it is considered to be profoundly associated with the macrophage in terms of inducing accumulation of cholesterol and the onset and the development of a lesion of arteriosclerosis.

As mentioned above, methods are being developed for the determination of a denatured lipoprotein with a view to allowing diagnosis of circulatory diseases typified by arteriosclerosis.

Specifically, a method for determining an oxidized lipoprotein by the use of an antibody capable of recognizing phosphatidylcholine oxide (JP-A-08-304395 and JP-A-09-288106), a method for determining an oxidized HDL by the use of 9F5-3a which is an antibody capable of recognizing oxidized phospholipid of HDL (particularly rhizophosphatidylcholine) and an anti-Apo-AI antibody (JP-A-09-33525), a method for determining an oxidized LDL by combining an antibody capable of recognizing all the LDL's which have undergone such chemical treatments as acetylation, conversion into malondialdehyde, and oxidation and an oxidizing agent (JP-A-09-5323), a method for determining a denatured or modified lipoprotein (a) by the use of an antibody which shows no cross-reacting property to plasminogen and LDL and is capable of recognizing a specific peptide that is suffered the structure, composition or configuration change thereof to be varied when the primary structure, higher order structure, or spatial structure is altered, decomposed, or chemically modified as by the oxidation, reduction, and decomposition such as hydrolysis, heating, or denaturation by the use of such a modifying agent as a denaturing agent of the lipoprotein (a) (JP-A-09-297137), and a method for determining a denatured lipoprotein by the combined use of an antibody against a lipoprotein modified with malondialdehyde and a surfactant (JP-A-08-101195) have been reported to date. None of them, however, has brought fully satisfactory effects.

### Disclosure of the Invention

An object of the present invention is to provide a method for the quantitative determination of a denatured lipoprotein in a biological sample and a method for the detection of cardiovascular disease. Also described herein is a reagent for the determination of a denatured lipoprotein, and a reagent for the detection of cardiovascular disease (not claimed).

In a first aspect, the invention provides a method for the quantitative determination of a denatured lipoprotein in a biological sample, which comprises contacting the biological sample with an antibody against phosphocholine and determining a formed immune complex.

In a second aspect, the invention provides a method for the quantitative determination of a denatured lipoprotein (a) in a biological sample, which comprises contacting the biological sample with an antibody against phosphocholine and an antibody against an apoprotein (a) and determining a formed immune complex.

The method of the first and second aspects may be for the detection of cardiovascular disease. Other preferred features of the invention are set out in the dependent claims.

Also described herein are the following items (21) to (36).
(21) A reagent for the quantitative determination of a denatured lipoprotein, which comprises an antibody against phosphocholine.
(22) A reagent for the quantitative determination of a denatured lipoprotein, which comprises an antibody against phosphocholine and an antibody against a lipoprotein.
(23) A reagent according to the above item (22), wherein the antibody against the lipoprotein is selected from the group consisting of an antibody against an Apo B protein, an antibody against an Apo A protein, and an antibody against an apoprotein (a).
(24) A reagent according to any of the above items (21) to (23), which contains a cryoprotective agent.
(25) A kit for the quantitative determination of a denatured lipoprotein, which comprises an antibody against phosphocholine.
(26) A kit for the quantitative determination of a denatured lipoprotein, which comprises an antibody against phosphocholine and an antibody against a lipoprotein.
(27) A kit according to the above item (26), wherein the antibody against the lipoprotein is selected from the group consisting of an antibody against an Apo B protein, an antibody against an Apo A protein, and an antibody against an apoprotein (a).
(28) A kit for the quantitative determination of a denatured lipoprotein according to any of the above items (25) to (27), which contains a cryoprotective agent.
(29) A reagent for the detection of cardiovascular disease, which comprises an antibody against phosphocholine.
(30) A reagent for the detection of cardiovascular disease, which comprises an antibody against phosphocholine and an antibody against a lipoprotein.
(31) A reagent according to the above item (30), wherein the antibody against the lipoprotein is selected from the group consisting of an antibody against an Apo B protein, an antibody against an Apo A protein, and an antibody against an apoprotein (a).
(32) A kit for the detection of cardiovascular disease, which comprises an antibody against phosphocholine.
(33) A kit for the detection of cardiovascular disease, containing an antibody against a phosphocholine and an antibody against a lipoprotein.
(34) A kit according to the above item (33), wherein the antibody against the lipoprotein is selected from the group consisting of an antibody against an Apo B protein, an antibody against an Apo A protein, and an antibody against an apoprotein (a).
(35) A kit for the detection of cardiovascular disease according to any of the above items (32) to (34), which comprises a cryoprotective agent.
(36) A cryoprotective agent for a biological sample, which comprises as an active component thereof a compound selected from the group consisting of sugar, high molecular substances, and hydrophilic organic solvents.

As the denatured lipoprotein, any lipoprotein may be included so long as it has undergone denaturation. Examples of the denatured lipoprotein include such denatured lipoproteins which have undergone chemical changes such as glycosylated denatured lipoprotein, oxidized denatured lipoprotein, acetylated denatured lipoprotein, and denatured protein undergone aldehyde conversion by the auction of malondialdehyde, etc., and 4-hydroxy-2-nonenal-modified denatured lipoprotein, denatured lipoproteins which have undergone morphological changes such as aggregation and binding induced by oxidation, conversion into thiol, treatment with enzyme such as lipoprotein lipase, physical treatment such as vortex; and temperature treatment such as freezing, and denatured lipoproteins which have undergone structural changes as the change of surface structure and the change of three-dimensional structure. Further, lipoproteins which show biological changes such as changes in electric charge, changes in molecular weight, changes in the affinity for a biological receptor, and changes in the ease of incorporation into a cell like macrophage as compared with the native lipoprotein are also included in the denatured lipoproteins.

The method for the quantitative determination of a denatured lipoprotein of the present invention does not need to be particularly discriminated so long as the method comprises immunologically determining the denatured lipoprotein by using an antibody against phosphocholine. The process in the determination method comprises a step of contacting the biological sample with an antibody against phosphocholine and a step of determining the amount of a denatured lipoprotein-anti-phosphocholine antibody complex formed by the reaction (immune complex).

As the determination method, the methods of immunological determinations may be mentioned. As the methods of immunological determinations, any method may be employed so long as it is a method utilizing antigen-antibody reactions such as immunoassay, immunoblotting, aggregation, complement fixation, hemolysis, precipitation, gold colloid method, chromatography; and immune staining, among which the immunoassay is preferred.

The immunoassay is a method of detecting or quantitatively determining an antibody or an antigen by using a labeled antigen or a labeled antibody. Depending on the method of labeling an antigen or an antibody, there are various types of the immunoassay such as radioimmunoassay (RIA), enzyme immunoassay (EIA or ELISA), fluorescent immunoassay (FIA), luminescent immunoassay, physicochemical detection (TIA, LAPIA, PCIA), and flow cytometry, among which the enzyme immunoassay is preferred.

As the radioactive label to be used in the radioimmunoassay, any of known radio-isotopes [Method of Biochemical Experiments, 15, Method for immunochemical identification, Written by J. Clausen and published by Tokyo Kagaku Dojin (1993), translated by Taiji Kato and Keiko Totani under supervision of Minoru Sasaki] can be used. For example, ³²P, ¹²⁵I, ¹³¹I, etc. may be used.

As the enzyme label for the use in the enzyme immunoassay, any of known enzymes (Method of Enzyme immunoassay, complied by Eiji Ishikawa et al. and published by Igaku Shoin) can be used. For example, alkaline phosphatase, peroxidase, luciferase, etc. may be used.

As the luminescent label for the use in the luminescent immunoassay, any of known luminophors [Bioluminescence and Chemoluminescence, Clinical Test 42 (1998), complied by Kazuhiro Imai andpublishedbyHirokawa Shoten] can be employed. For example, acrydinium esters, lophine, etc. may be used.

As the fluorescent label for the use in the fluorescent immunoassay, any of known fluorescent materials (Method of Fluorescent Antibody, written by Akira Kawaoi and published by Soft Science Corp.) can be employed. For example, FITC, RITC, etc. may be used.

As the method of determination in the immunoassay method, the method of competitive assay and the sandwich method [Immunology Illustrated, fifth edition, published by Nankodo] may be mentioned.

Specifically, a denatured lipoprotein in a biological sample can be detected or determined by contacting the biological sample with an antibody against phosphocholine (hereinafter referred to as "anti-phosphocholine antibody") and then allowing the sample to react with an antibody capable of reacting with the anti-phosphocholine antibody having a label bound thereto. The denatured lipoprotein in the biological sample can also be detected or determined by contacting the biological sample with an anti-phosphocholine antibody having a label bound thereto thereby inducing the reaction therebetween.

As the antibody for the use in the immunoassay as mentioned above, either a polyclonal antibody or a monoclonal antibody can be used, an antibody fragment such as Fab, Fab', and F(ab)₂ can also be used. As the monoclonal antibody, any monoclonal antibody may be used, for example, an antibody spontaneously produced in a living organism, an antibody produced from a hybridoma obtained by using a non-human animal, a genetically engineered antibody capable of expressing an antibody molecule by using a gene engineering technique based on a DNA which codes for an amino acid sequence of a variable region and a constant region of each of heavy chain and light chain forming an antibody molecule, and the like.

The anti-phosphocholine antibody to be used in the present invention may be any of the antibodies as mentioned above, so long as it reacts with phosphocholine. The examples are T-15 antibody {J. Exp. Med., 132, 737 (1970)], monoclonal antibody KTM-285 produced by hybridoma KTM-285 (FERM BP-7589) and genetically engineered antibody KTM-2001 produced by transformant KTM-2001 (FERM BP-7549).

The present invention further relates to a method for the immunological determination of a denatured lipoprotein by the use of an antibody against phosphocholine and an antibody against a lipoprotein.

Specifically, the denatured lipoprotein in a biological sample can be detected or determined by fixing a first antibody (primary antibody) on a solid phase, then contacting the biological sample with the first antibody, washing out unaltered sample components in the sample, and allowing a second antibody (secondary antibody) to react with an immune complex of a target substance in the biological sample and the first antibody.

The examples of the antibodies which can be used as the primary antibody and the secondary antibody are an anti-phosphocholine antibody and an anti-lipoprotein antibody. As regards the combination of the primary antibody and the secondary antibody, the combination of an anti-phosphocholine antibody as the primary antibody and an anti-lipoprotein antibody as the secondary antibody is preferred.

The term "lipoprotein" refers to a substance existing in blood in the form of a structure in which lipids such as cholesterol ester and triglyceride form cores and the surfaces of these cores are covered with phospholipid and free cholesterol and further with proteins (apoprotein) as well.

The examples of the lipoprotein are very low density lipoprotein (hereinafter abbreviated as "VLDL"), low density lipoprotein (hereinafter abbreviated as "LDL"), high density lipoprotein (hereinafter abbreviated as "HDL"), intermediate density lipoprotein (hereinafter abbreviated as "IDL"), and lipoprotein (a) (hereinafter abbreviated as "Lp(a)").

The VLDL is a lipoprotein in the form of particles having a specific gravity in the range of 0.96 to 1. 006 which comprises triglyceride as main lipid and proteins such as Apo B-48, Apo C and Apo E, and functions as a carrier for endogenous fat.

The LDL is a lipoprotein in the form of particles having a specific gravity in the range of 1.006 to 1.063 which is rich in cholesterol, comprises Apo B-100 as a main protein and functions to carry cholesterol from a liver to peripheral tissues.

The HDL is a lipoprotein in the form of particles having a specific gravity in the range of 1.063 to 1.21, which comprises phospholipid and cholesterol as main lipids and Apo A-I, Apo A-II, Apo C, etc. as main proteins, and functions to carry cholesterol out of peripheral tissues to metabolize it in a liver.

The IDL is an intermediate metabolite occurring during the conversion from VLDL to LDL in the form of particles having a specific gravity in the range of 1.006 to 1.019, which is rich in cholesterol more than the VLDL, and comprises Apo B-100, Apo C, Apo E, etc. as main proteins.

The Lp (a) is a lipoprotein in the form of particles having a specific gravity in the range of 1.03 to 1.08, in which an apoprotein (a) of an unique structure is bound to lipoprotein particles similar to LDL, and which has function associated with the coagulation of blood.

Accordingly, for the antibody against various types of lipoproteins, a main apoprotein contained in each of the various lipoprotein may be used. By using an anti-phosphocholine antibody and an antibody capable of reacting with the main apoprotein contained in the lipoprotein (hereinafter referred to occasionally as "anti-apoprotain antibody"), it is possible to detect and determine various denatured lipoproteins.

Examples of the antibody capable of reacting with an apoprotein contained in a denatured lipoprotein are anti-Apo-AI protein antibody, anti-Apo-AII protein antibody, anti-Apo-AIV protein antibody, anti-Apo-B-100 protein antibody, anti-Apo-B-48 protein antibody, anti-Apo-CI protein antibody, anti-Apo-CII protein antibody, anti-Apo-CIII protein antibody, anti-Apo-D protein antibody, anti-Apo-E protein antibody.

When a denatured LDL is to be detected or determined as a denatured lipoprotein, for example, an anti-Apo-B protein antibody may be used as an anti-apoprotein antibody. When a denatured VLDL is to be detected or determined, an anti-Apo-E protein antibody may be used as an anti-apoprotein antibody. When a denatured HDL is to be detected or determined, an anti-Apo-AI protein antibody may be used as an anti-apoprotein antibody. When a denatured Lp(a) is to be detected or determined, an anti-apoprotein (a) antibody is used as an anti-apoprotein antibody.

As described above, by using an antibody against a specific lipoprotein, it is possible to determine or detect a denatured lipoprotein of a specific type. Further, this method is not only capable of determining or detecting a specific denatured lipoprotein but also capable of determining or detecting two or more types of denatured lipoproteins simultaneously by combining several anti-apoprotein antibodies at the same time.

Specifically, by using an antibody against the HDL, namely an anti-Apo-AI protein antibody, and an antibody against the LDL, namely an anti-Apo-B protein antibody, it is possible to determine or detect a denatured HDL and a denatured LDL.

The method for determining various type of denatured lipoprotein is alternatively enabled to determine or detect a specific type of denatured lipoprotein by preparatorily purifying a biological sample as by the treatment of centrifugal separation and then causing an antibody against phosphocholine to react with the purified sample.

Examples of the method for purifying the LDL by centrifugally separating a biological sample are a method for taking out an LDL fraction by subjecting an normal human serum to density gradient centrifugation using ethylene diamine sodium tetraacetate (EDTA-2Na) and NaBr (JP-A-07-238098), a method for taking out an LDL fraction by adding EDTA to human plasma obtained from heparinized blood, centrifuging the resultant mixture, further adding a KBr solution to the residue, and again centrifuging the mixture (JP-A-08-304395), and a method for recovering LDL from human plasma by ultracentrifugation (JP-A-09-288106).

An example of the method for purifying Lp(a) from a biological sample by centrifugal separation is a method which comprises adding EDTA to a heparinized human plasma, superposing an EDTA-containing NaCl solution thereon centrifuging the mixture, adding a KBr solution to the residue, again centrifuging the mixture, subjecting the resultant solution to gel filtration, and passing the result of the filtration through a column packed with lysine Sepharose B thereby obtaining an Lp(a) fraction (JP-A-08-304395).

As a means to purify HDL by centrifugal separation, mentioned is a method which comprises adding sodium tetraacetate (EDTA-2Na) and NaCl to human serum to adjust the density of the solvent at 1.063, centrifuging the resultant solution, adding NaBr to the resultant solution to adjust the density of the solvent at 1.21 and centrifuging the mixture with the resultant solution thereby obtaining a HDL fraction [Course of New Biological Experiments, Lipid I, 195 (1993), written by Taku Yamamura and published by Tokyo Kagakudojin].

In the method for determination according to the present invention, a receptor for an apoprotein, an apoprotein-binding protein, etc. may be used in place of the anti-lipoprotein antibody. As the substance usable for this replacement, lecithin-cholesterol-acyl transferase capable of recognizing Apo-AI, LDL receptor of cells of liver and small intestine capable of recognizing Apo-B-100, lipoprotein lipase capable of recognizing Apo-CII, E receptor of liver capable of recognizing Apo-E, etc. may be mentioned.

As a typical example, the immunoassay using an anti-phosphocholine antibody and an anti-Apo B protein antibody will be described below.

An anti-phosphocholine antibody-bound solid phase is prepared by adding a solution having an anti-phosphocholine antibody diluted with a buffer to a solid phase such as a 96-well microplate, incubating the solution therein at a low temperature for 1 to 24 hours, discarding the solution, adding a buffer such as Tris-HCl (pH 8.0) containing BSA at a concentration of about 1% (W/V) to the solid phase, incubating the solution at room temperature for 1 to 12 hours thereby blocking, and thereafter washing the solid phase with PBS (pH 7.4) containing a surfactant such as Tween 20 at a concentration of 0.05% (V/V).

Then, a biological sample such as plasma is diluted with a sample-diluent such as a 10 mmol/L phosphate buffer (pH 7.4) containing 1% BSA, 5% polyether, and 140 mmol/L NaCl. The diluted sample is added to the wells, incubated therein at room temperature for 1 to 12 hours, and then washed with PBS (pH 7.4) containing 0.05% (v/v) Tween 20.

Further, an anti-human Apo B protein antibody diluted with PBS (pH 7.4) containing 2% (v/v) BSA and labeled with peroxidase is added as a secondary antibody to each of the wells and incubated therein at room temperature for 10 to 60 minutes. Subsequently, the wells are washed with PBS (pH 7.4) containing 0.05% (v/v) Tween 20 and subjected to determination of label. The determination of the denatured LDL in the sample can be attained based on a calibration curve prepared in advance by using standards of known concentration. For the detection of peroxidase, for example, a 3,3',5,5'-tetramethyl benzidine (TMBZ) solution can be used.

By this assay, the denatured LDL can be determined. The present invention also concerns a method for the detection of cardiovascular disease, which comprises contacting the biological sample with an antibody against phosphocholine and determining a produced immune complex.

Examples of the cardiovascular diseases are various circulatory diseases including diseases of coronary system such as myocardial infarction and angina pectoris, diseases of cerebral artery system such as cerebral infarction and cerebral vascular dementia, diseases of renal artery such as nephropathy and diabetic nephropathy, and diseases of peripheral artery such as peripheral artery occlusion.

In the present invention, cardiovascular disease can be detected by determining a denatured lipoprotein, that is determining the denatured lipoprotein in a biological sample by using an anti-phosphocholine antibody or by using both of an anti-phosphocholine antibody and an antibody against lipoprotein.

As the antibody against lipoprotein to be used in the determination mentioned above, an antibody against an apoprotein constituting a denatured lipoprotein may be mentioned, examples of which include an antibody against an Apo-B protein constituting a denatured LDL, an antibody against an Apo-E protein constituting a denatured VLDL, an antibody against an Apo-AI protein constituting a denatured HDL, and an antibody against an apoprotein (a) constituting a denatured Lp(a). By combining these antibodies, it is possible to determine various denatured lipoproteins and detect cardiovascular disease.

The concentration of a denatured lipoprotein contained in the biological sample of a patient of the circulatory disease shows a significant rise as compared with that of the denatured protein contained in the biological sample of a healthy person. Thus, by setting a cutoff value on the denatured lipoprotein, it is possible to detect circulatory disease in a patient when the biological sample taken therefrom is tested for the amount of a denatured lipoprotein and the numerical value of the determination exceeds the cutoff value.

The value of the denatured lipoprotein in a healthy person can be obtained by taking a biological sample from the healthy person who has been in advance confirmed clinically to suffer from no circulatory disease and determining the denatured lipoprotein in the biological sample by the present method for determining a denatured lipoprotein.

The method for clinically confirming various circulatory disease is not particularly limited, and examples of the method include a method of coronary angiography, a method of mechanical diagnosis using a load electrocardiogram and echocardiography, and a method for effecting the confirmation from the subjective symptoms such as pectoralgia.

The term "cutoff value" as used herein means the numerical value which is fixed with respect to a certain substance to discriminate a group of target diseases and a group of non-disease. When target diseases and non-diseases are rated, the diseases can be rated by taking a disease registering a numerical value not exceeding the cutoff value as negative and a disease registering a numerical value exceeding the cutoff value as positive or by taking a disease registering a numerical value not exceeding the cutoff value as positive and a disease registering a numerical value exceeding the cutoff value as negative (Abstract of Methods of Clinical Examinations, compiled by Masamitsu Kanai and published by Kimbara Shuppan K.K.).

As indices to be used for the purpose of evaluating the clinical utility of the cutoff value, sensitivity and specificity may be mentioned.

When a certain population is rated by using the cutoff value, the numerical value represented by a/(a + b) can be expressed as sensitivity (true positive ratio) and the numerical value represented by d/(c+d) as specificity (true negative ratio), wherein "a" denotes those of the patients of disease who are rated as positive (true positive), "b" denotes those of the patients of disease who are nevertheless rated as negative (false-negative), "c" denotes those of the non-patients of disease who are nevertheless rated as positive (false-positive), and "d" denotes those of the non-patients of disease which are rated as negative (true negative).

The distributions of the numerical values of the determination of the group of target diseases and the group of non-diseases generally overlap partly. By raising or lowering the cutoff value, therefore, the sensitivity and the specificity are changed. By lowering the cutoff value, the sensitivity is heightened and the specificity is lowered. By heightening the cutoff value, the sensitivity is lowered and the specificity is heightened. For the sake of the method of rating, it is favorable that the numerical values of both sensitivity and specificity are high. The method of rating using sensitivity and specificity whose numerical values do not exceed 0.5 cannot be recognized as useful.

As the method of setting the cutoff value, mentioned are a method which sets any numerical value of either of the opposite ends from the center including 95% of the distribution of the group of non-diseases as the cutoff value and a method which sets the average value + twice the standard deviation (SD) or the average value - 2SD as the cutoff value when the group of non-diseases shows a normal distribution.

In the present invention, any biological sample may be used, for example, blood, urine, spinal fluid, and punctured fluid, among which blood is particularly favorable. Examples of the blood are whole blood, plasma, serum, hemolysate, endoglobular fluid. Among other blood components cited above, serum or plasma is particularly advantageous.

As the reagent for the use in the determination of a denatured lipoprotein and the reagent for the use in the method for the detection of cardiovascular disease, reagents containing an anti-phosphocholine antibody may be mentioned. The reagent may further contain an antibody against a lipoprotein such as an anti-apoprotein antibody. The reagent which contains an anti-phosphocholine antibody and an antibody against lipoprotein is capable of fractionally determining or detecting various denatured lipoproteins. The anti-phosphocholine antibody or the anti-apoprotein antibody may be an antibody which has a label bound thereto.

As the anti-phosphocholine antibody, any of the antibodies mentioned above can be used so long as it has a nature of reacting with phosphocholine. As the anti-phosphocholine antibody, T-15 antibody [J. Exp. Med., 132, 737 (1970)], monoclonal antibody KTM-285 produced by hybridoma KTM-285 (FERM BP-7589), and genetically engineered antibody KTM-2001 produced by transformant KTM-2001 (FERM BP-7549) may be mentioned.

As the antibody against lipoprotein, the antibodies against various apoproteins such as Apo-AI, Apo-AII, Apo-AIV, Apo-B-100, Apo-B-48, Apo-CI, Apo-CII, APO-CIII, Apo-D, and Apo-E may be mentioned.

The kit described herein is constituted by the combination of instruments or reagents. A kit which includes substances essentially identical with the component elements described below or substances essentially identical with part thereof is embraced in the kit even when it differs structurally or morphologically.

The reagents contain only an anti-phoshocholine antibody or both of an anti-phosphocholine antibody and an anti-lipoprotein antibody and if necessary, the reagents further include standards such as a diluent for a biological sample, a solid phase for fixing an antibody, a reaction buffer, a surfactant, a labeled secondary antibody or an antibody fragment thereof, a reagent for the detection of a label, and a denatured lipoprotein. A standard may be bound to the anti-phosphocholine antibody or the anti-lipoprotein antibody.

As the diluent for a biological sample, the aqueous solutions of a surfactant and a buffer which contain proteins such as BSA and casein may be mentioned.

As the solid phase for fixing an antibody, the solid phase which has an anti-phosphocholine antibody or an anti-lipoprotein antibody or an antibody fragment thereof fixed on the material obtained by shaping various high molecular material to suit the purpose of use is used. As the shape of the solid phase, tube, bead, plate, fine particles like latex, and stick may be mentioned. As the material, high molecular materials such as polystyrene, polycarbonate, polyvinyl toluene, polypropylene, polyethylene, polyvinyl chloride, nylon, polymethacrylate, gelatin, agarose, cellulose and polyethylene terephthalate, glass, ceramics, and metals may be mentioned. As the method for fixing an antibody on a solid phase, known methods such as physical methods, chemical methods, and methods combining them are applicable for the preparation of the solid phase. For example, a method wherein an antibody or an antibody fragment has been fixed on a solid phase hydrophobically on a 96-well immunoassay grade mictotiter plate made of polystyrene may be mentioned.

Any of reaction buffers can be used so long as it is capable of providing a solvent environment for reacting and binding the antibody on an antibody fixing solid phase with an antigen in a biological sample. Examples are surfactants, buffer agents, proteins such as BSA and casein, antiseptics, stabilizers, reaction accelerators, etc.

As the labeled secondary antibody or the antibody fragment thereof, the antibody or the antibody fragment to be used in the present invention which has been labeled with a labeling enzyme such as horseradish peroxidase (HRP), bovine intestine alkaline phosphatase, and β-galactosidase or its mixture with a buffer agent, a protein such as BSA and casein, and an antiseptics is used.

The reagent for the detection of a labeled antibody to be used varies based on the labeling enzyme. For example, when the labeling enzyme is horseradish peroxidase, substrates for the determination of absorbance such as tetramethyl benzidine and orthophenylene diamine, fluorescent substrates such as hydroxyphenyl propionic acid and hydroxyphenyl acetic acid, and luminescent substrates such as luminol are used, and when the labeling enzyme is alkaline phosphatase, substrates for the determination of absorbance such as 4-nitropheyl phosphate and fluorescent substrates such as 4-methylunbelliphenyl phosphate are used as the reagent for the determination of a labeled antibody.

As the standard, denatured lipoproteins obtained by a method which comprises isolating and purifying a lipoprotein by an ultracentrifugation technique and oxidizing the purified lipoprotein with a metallic ion such as copper ion, acetylating the purified lipoprotein by the reaction with acetic anhydride, or causing the purified lipoprotein to react with malondialdehyde,denatured lipoproteins to be coagulated by freezing and thawing, and denatured lipoproteins denatured by physical vibration are available.

It is preferable that the determination of a denatured lipoprotein in a biological sample is carried out immediately after the collection of the biological sample, however, a preserved biological sample may be subjected to the determination. The method for preserving a biological sample is not particularly restricted, so long as the sample is preserved under the conditions that the amount of the denatured lipoprotein is not changed. For example, it is preferred to be carried out at a low temperature such as 0 to 10°C which avoids freezing the sample in a dark place in the absence of vibration.

The biological sample to be used in the present invention may have been preserved by freezing. The freezing of the biological sample is preferred to be carried out in the presence of the cryoprotective agents mentioned herein below.

Examples of the cryoprotective agent are sugars such as sucrose, trehalose, lactose, mannitol, and glucose, high molecular substances such as dextran, dextran sulfate, pullulan, polyethylene glycol, and carboxymethyl cellulose, and water-soluble organic solvents such as glycerol and dimethyl sulfoxide. These substances may be used in an amount of 0.1 to 50%, preferably 1 to 30%, and more preferably 5 to 20% in the biological sample.

The freezing is carried out with keeping the temperature of the solvent below the freezing point. The fall of the temperature is preferred to advance quickly from the temperature for starting supercooling to the temperature of supercooling and recover instantaneously the latent heat of freezing. It is preferred tobe effected by the use of a program freezer. For example, the solvent is cooled to 10°C, a temperature for starting the cooling, then cooled from this temperature to the neighborhood of -5 to -10°C, a temperature for starting supercooling at a rate of - 1°C/min., and cooled suddenly from this temperature to - 40°C, a supercooling temperature. Subsequently, if necessary, the solvent may be quickly raised to -18°C, a temperature for retaining a spontaneously elevated temperature, and thereafter cooled to the target temperature at a rate of -1°C/min. Alternatively, the solvent may be cooled from -40°C, a temperature for supercooling, to the target temperature at a rate of - 1°C/min. without being brought to the temperature for retaining the spontaneously elevated temperature. The rate of lowering to the freezing temperature is preferably not lower than -0.1°C/min. and more preferably not lower than -1.0°C/min. The temperature to be finally reached is preferred to be not higher than -20°C, more preferably not higher than -30°C, and particularly preferably -196°C which is attainable with liquid nitrogen.

In the determination of a denatured lipoprotein in a biological sample preserved by freezing, though the method for thawing the frozen sample does is not particularly restricted, the thawing may be accomplished by a method which comprises shaking the frozen sample and keeping it in a cold water bath or a hot water bath, for example.

The reagent and the kit for determining a denatured lipoprotein and the reagent and the kit for detecting cardiovascular disease may include the cryoprotective agent as described above (not claimed).

Now, an example of the method for producing a monoclonal antibody will be described in detail below.

### 1. Method for production of monoclonal antibody

### (1) Preparation of antigen

As the antigen, though phosphocoline may be directly used as an immunogen, it is preferable to use phosphocoline to which some other high molecular substance is bound as an immunogen. As the high molecular substance, bovine serum albumin, proteins, polysaccharides, nucleic acids, and synthetic high molecular substances may be mentioned, and lipoproteins are preferable. The phosphocholine is preferred to be in the form of a phosphocholine derivative which comprises phosphocholine and some other compound, for example. The phosphocholine derivative is not particularly restricted, so long as a phosphocholine group in the phosphocholine derivative is recognized as an epitope. As the phosphocholine derivative, 1-palmitoyl-2-(9-oxononanoyl)-glycero-3-phosphocholine and 1-palmitoyl-2-(5-oxovaleroyl)-glycero-3-phosphocholine may be mentioned. These phosphocholine derivatives are prepared by the method described in the literature [J. Biol. Chem., 266(17), 11095 (1991)].

As the lipoprotein as a high molecular substance, LDL, HDL, VLDL, IDL, etc. may be mentioned, and LDL is preferable. The LDL is not particularly restricted and includes LDL derived from blood, egg yolk, milk, etc. of various animals such as human, horse, cattle, rabbit, dog, goat, and sheep. Among the above mentioned LDL, the LDL from human blood is preferable. The ligation of a phosphocholine derivative on a lipoprotein can be accomplished by mixing them together by causing the phosphocholine derivative to be added dropwise into a solution of the lipoprotein.

### (2) Immunization of animal and preparation of antibody-producing cell

A mouse, rat, hamster, rabbit, guinea pig, goat, sheep, or chicken of 3 to 20 weeks old is immunized with an antigen prepared in the (1) above, and the antibody-producing cells are collected from spleen, lymphoglandula, and peripheral blood of the animal. In the case of producing a polyclonal antibody, it is preferred to use a rabbit, guinea pig, goat, sheep, and chicken. For the production of a monoclonal antibody, it is preferred to use a mouse and rat.

The immunization is carried out by injecting an antigen together with a suitable adjuvant (for example, complete Freund's adjuvant or aluminum hydroxide gel plus pertussis vaccine) subcutaneously, intravenously, or intraperitoneally into a given animal. When the antigen is partial peptide, a conjugate is produced with a carrier protein such as BSA (bovine serum albumin) and KLH (keyhole limpet hemocyanin), and the conjugate thus obtained is used as an immunogen.

The administrations of the antigen may be carried out three to ten times at an interval of one to two weeks after the first immunization. On the third to seventh day after each administration, blood of the animal is taken via ophthalmic venous plexus and then the reaction of the serum with the antigen is examined by enzyme immunoassay [Antibodies - A Laboratory Manual, Cold Spring Harbor Laboratory, 1988]. The mouse, rat, or hamster whose serum shows a fully satisfactory antibody titer is used as the source of the antibody-producing cell.

In the case of the polyclonal antibody, blood of an animal which has completed immunization is taken either periodically or at once by exsanguination. Generally, the blood is taken out without preventing coagulation, and the blood taken out is allowed to coagulate once and then centrifuged to recover a serum fraction, which is put to use. If necessary, the antibody in the blood may be purified and used. As the method for purification, a salting-out method with ammonium sulfate, for example, a method of ion-exchange chromatography, a method of gel filtration column chromatography, a method of affinity column chromatography using protein A or protein G, and a method of affinity column chromatography using a gel having an antigen fixed on a solid phase may be used either singly or in combination.

As the source for an antibody-producing cell for preparing a monoclonal antibody, spleen, lymphoglandula, and peripheral blood of an immunized animal may be mentioned. Alternatively, cells which have undergone the so-called in vitro immunization, i.e., a process which comprises taking out cells responsible for producing antibody from spleen, lymphoglandula, or peripheral blood of an animal which has not been immunized and immunizing the cells directly to convert them into an antibody-producing cells [Arai, Ota, Practical Medicine, 6, 43 (1988)], may be used.

To carry out the fusion of antibody-producing cells with myeloma cells, spleen is excised from immunized mouse, rat, or hamster on the third to seventh day after the final administration of the antigen substance, and spleen cells are collected from the excised spleen. The spleen cells to be supplied for the fusion can be obtained by mincing the spleen in MEM culture medium (manufactured by Nissui Seiyaku K.K.), loosing the minced spleen with forceps, centrifuging (1200 rpm, for five minutes), discarding a supernatant, treating the residue with a Tris-ammonium chloride buffer (pH 7.65) for one to two minutes to remove erythrocytes, and washing three times with MEM culture medium.

### (3) Preparation of myeloma cells

As the myeloma cells, a cell strain derived from a mouse are used. For example, 8-azaguanine-resistant mouse (derived from BALB/c) myeloma cell strain P3-X63Ag8-U1) [Current Topics in Microbiology and Immunology, 18, 1-7 (1978)], P3-NS1/1-Ag41 (NS-1) [European J. Immunology, 6, 511-519 (1976)], SP2/0-Ag14(SP-2) [Nature, 276, 269-270 (1978)], P3-X63-Ag8653(653) [J. Immunology, 123, 1548-1550 (1979)], and P3-X63-Ag8(X63) [Nature, 256, 495-497 (1975)] may be used. These cell strains are subcultured with a 8-azaguanine culture medium [a culture medium obtained by preparing a culture medium (hereinafter referred to as "normal culture medium") by adding glutamine (1.5 mmoles/L), 2-mercapto ethanol (5 x 10⁻⁵ moles/L), gentamicin (10 µg/mL), and fetal calf serum (FCS) to an RPMI-1640 medium and further adding 8-azaguanine (15 µg/mL) to the normal culture medium]. The cell strain, three to four days before the fusion of cells, is subcultured in a normal culture medium to secure a cell number of not less than 2 x 10⁷ on the day of fusion.

### (4) Fusion of cells

The fusion of cells was invented by Kohler and Milstein [Nature, 256, 495 (1975)] and has been quickly developed. In the present invention, variously improved methods are available for the fusion of cells.

The antibody-producing cells immunized in the (2) above and the myeloma cells obtained in the (3) above are thoroughly washed with MEM culture medium or PBS (disodium phosphate 1.83 g, monopotassium phosphate 0.21 g, sodium chloride 7.65 g, and distilled water 1 liter, pH 7.2), mixed to adjust the cell number at the ratio of antibody-producing cells : myeloma cells = 5 to 10 : 1, centrifuged (1, 200 rpm, for five minutes), and the supernatant is discarded. The settled cells are thoroughly loosened, and a mixed liquid of 2 g of polyethylene glycol 1,000 (PEG-1,000), 2 ml of MEM, and 0.7 ml of dimethyl sulfoxide is added thereto with stirring in an amount of 0.2 to 1 mL/10⁸ antibody-producing cells at 37°C, and 1 to 2 mL of MEM culture medium is added several times at an interval of one to two minutes, and MEM culture medium is added till the total volume reaches 50 mL. The resultant mixture is centrifuged (900 rpm, for five minutes) and the supernatant is discarded. The resultant cells are gently loosened, and gently suspended in 100 ml of a HAT culture medium [a culture medium prepared by adding hypoxanthine (10⁻⁴ moles/L), thymidine (1.5 x 10⁻⁵ moles/L), and aminopterin (4 x 10⁻⁷ moles/L) to a normal culture medium] by sucking and spouting out a measuring pipette. The suspension is dispensed in an 96-well culturing plate in an amount of 100 µL/well and incubated in an 5% CO₂ incubator at 37°C for 7 to 14 days.

After the completion of culture, part of the supernatant is taken and tested by enzyme immunoassay which will be specifically described herein below to select cells which react with phosphocholine and avoid reacting with an antigen containing no phosphocholine. Then, the cells are cloned twice by limiting dilution [the first cycle using a HT culture medium (the culture medium prepared by removing aminopterin from an HAT culture medium) and the second cycle using a normal culture medium]. The cells which show a high antibody titer steadily are selected as an anti-phosphocholine monoclonal antibody-producing hybridoma strain.

### Method of enzyme immunoassay

An antigen or a cell expressing an antigen is coated on a 96-well plate and is allowed to react with a hybridoma culture supernatant or a purified antibody as the first antibody.

After the reaction with the first antibody, the plate is washed and a second antibody is added to the plate.

The term "second antibody" as used herein means an antibody obtained by labeling an antibody capable of recognizing immunoglobulin of the first antibody with biotin, an enzyme, a chemical luminescent substance, or a radioactive compound. Specifically, when a mouse is used in the preparation of hybridoma, an antibody capable of recognizing mouse immunoglobulin is used as the second antibody.

After the reaction, the second antibody is subjected to reaction proper to the substance which has labeled with the second antibody and is selected as a hybridoma which produces a monoclonal antibody specifically reacting with the antigen

### (5) Preparation of monoclonal antibody

To a mouse or nude mouse of 8 to 10 weeks old which have subjected to pristane treatment (which comprises intraperitoneally administering 0.5 ml of 2,6,10,14-tetramethyl pentadecane (pristane) to a mouse and breeding it for two weeks), monoclonal antibody-producing hybridima cells obtained in the (4) above are intraperitoneally administered at a dose of 2 x 10⁶ to 5 x 10⁷ cells/animal. In 10 to 21 days, the hybridoma forms ascites carcinoma in the mouse. The ascites is collected from the mice, centrifuged (3,000 rpm, for five minutes) to remove the solid matter, salted out with 40 to 50% ammonium sulfate, and purified by sedimentation with caprylic acid, DEAE-Sepharose column, protein A-column, or gel filtration column to collect an IgG or IgM fraction, which is used as a purified monoclonal antibody.

The sub-class of an antibody is determined by enzyme immunoassay using a sub-class typing kit. The amount of the protein is determined by Lowry method and calculated from the absorbance at 280 nm.

As the hybridoma which is produced by the method described above and which produces a monoclonal antibody used for the method of the present invention, the hybridoma KTM-285 is mentioned. The hybrodoma KTM-285 has been deposited with International Patent Organism Depositary, National Institute, Advanced Industrial Science and Technology, located at AIST Tsukuba Central 6, 1-1-1 Higashi, Tsukuba, Ibaraki, Japan (Postal No. 305-8566) on May 16, 2001 under Accession No. FERM BP-7589. Hereinafter, the monoclonal antibody produced by the hybridoma KTM-285 will be simply referred to as "KTM-285 antibody". 2. Method for production of humanized antibody (1) Construction of vector for expressing humanized antibody

The monoclonal antibody used in the method of the present invention, besides being acquired in accordance with the method of production of a monoclonal antibody described in 1 above, can be prepared by obtaining cDNA coding for a heavy chain (H chain) and a light chain (L chain) of the antibody by using a genetic engineering technique from the cells as mentioned above, constructing a recombinant vector in which cDNA coding for the H chain and the L chain is inserted into the downstream of a promoter of the vector for expressing an antibody, and culturing in a suitable culture medium the antibody expressing cells obtained by introducing the recombinant vector in host cells or administering the recombinant vector to form ascites carcinoma in the animal, and separating and purifying the culture solution or the ascites. When the monoclonal antibody produced by the hybridoma is a multimer such as the IgA type, the IgM type, etc., the antibody of the IgG type can be obtained by producing an antibody in accordance with the method described in the present paragraph.

The vector for expressing an antibody as described above is an expression vector for an animal cell having incorporated therein a gene coding for a constant region heavy chain (CH) and a constant region light chain (CL) which form a constant region of a suitable animal antibody, and can be constructed by inserting genes coding for CH and CL of the suitable animal antibody into the expression vector for an animal cell.

As the C region of an animal antibody, any of C regions of animal antibody such as Cγ1 or Cγ4 for human antibody H chain, Cγ1, Cγ2a, Cγ2b, or Cγ3 for mouse antibody H chain, Cκ for human antibody L chain, and Cκ for mouse antibody L chain. As the gene coding for C region of an antibody, a chromosome DNA which comprises exon and intron can be used, and cDNA can be used as well. As the expression vector for an animal cell, any vector that is capable of integrating and expressing a gene coding for C region of an antibody can be used.

Examples of the vector include pAGE107 [Cytotechnology, 3, 133 (1990)], pAGE103 [J. Biochem, 101, 1307 (1987)], pHSG274 [Gene, 27, 223 (1984)], pKCR [Proc. Natl. Acad, Sci., 78, 1527 (1981)], pSG1βd2-4 [Cytotechnology, 4, 173 (1990)]. As the promoter and enhancer used for the expression vector for an animal cell, early promoter and enhancer for SV40 [J. Biochem, 101, 1307 (1987)], LTR promoter and enhancer for mouse Moloney leukemia virus [Biochem. Bioplhys. Res. Comun., 149, 960 (1987)], and promoter for immonoglobulin H chain [Cell, 41, 479 (1985)] and the enhancer [Cell, 33, 717 (1983)] may be mentioned.

As the expression vector, either of the type having the H chain and the L chain of an antibody exist on different vectors or of the type having these chains exist on the same vector (tandem type) may be used. The expression vector of tandem type is preferably used in terms of easy construction of an expression vector, easy introduction into an animal cell, and good balance of the expressed amounts of the H chain and the L chain of an antibody in an animal cell [J. Immunol. Methods, 167, 271 (1994)]. As the vector of tandem type for expressing humanized antibody, pKANTEX93 (WO 97/10354) and pEE18 (Hybridoma, 17, 559-567, 1988) may be mentioned.

The vector for expressing humanized antibody thus constructed can be used for expressing a human chimeric antibody and a human CDR grafted antibody in an animal cell.

### (2) Preparation of DNA coding for variable region of antibody

cDNA coding for a variable region heavy chain (V) and a variable region light chain (VL) of an antibody such as a mouse anti-phosphocholine antibody can be obtained as follows. A cDNA library is prepared from cells producing a mouse anti-phosphocholine antibody which is a natural antibody such as mouse peripheral blood cells or mouse spleen cells by a conventional method [Molecular Cloning, 2nd edition, Cold Spring Harbor Lab. Press, New York (1989); hereinafter abbreviated as "Molecular Cloning 2nd edition") and the Current Protocols in Molecular Biology Supplement 1-38; hereinafter abbreviated as "Current Protocols")].

As the method for preparing a cDNA library, methods disclosed in Molecular Cloning 2nd edition (1989), Current Protocols in Molecular Biology (1987), and DNA Cloning 1: Core Techniques, A Practical Approach, Second Edition, Oxford University Press (1995) and methods utilizing commercially available kits such as Superscript Plasmid System for cDNA Synthesis and Plasmid Cloning (manufactured by Gibco BRL Corp.) and ZAP-cDNA Synthesis Kit (manufactured by Stratagene Corp.) may be mentioned. Further, commercially available cDNA libraries such as a mouse spleen cell cDNA library manufactured by Takara Shuzo, Co., Ltd. can also be used.

As a cloning vector preparing a cDNA library, any of phage vector or plasmid vector, etc. can be used, so long as it is capable of performing autoreproduction in Escherichia coli K12 strain. Examples are ZAP Express [manufactured by Stratagene Corp, Strategies, 5, 58 (1992)], pBluescript II SK (+) [Nucleic Acids Research, 17, 9494 (1989)], λzap II (manufactured by StratageneCorp.), λgt10, λgt11 [DNA Cloning, A Practical Approach, 1, 49 (1985)], λ TriplEx (manufactured by Clontec Corp.), λ BlueMid (manufactured by Clontec Corp.), λ ExCell (manufactured by Pharmacia Corp.), pT7T318U (manufactured by Pharmacia Corp.), pcD2 [Mol. Cell. Biol., 3, 280 (1983)], and pUC18 (Gene, 33, 103 (1985)].

As the Escherichia coli used for introducing a vector having cDNA integrated, any microorganism can be used, so long as it belongs to genus Escherichia coli. Examples include Escherichia coli XL1-Blue MRF' [manufactured by Stratagene Corp., Strategies, 5, 81 (1992)], Escherichia coli C600 [Genetics, 39,440 (1954)], Escherichia coli Y1088 [Science, 222, 778 (1983)], Escherichia coli Y1090 [Science, 222, 778 (1983)], Escherichia coli NM522 [J. Mol. Biol., 166, 1 (1983)], Escherichia coli K802 [J. Mol. Biol., 16, 118 (1966)], and Escherichia coli JM105 (Gene, 38, 275 (1985)]. The cDNA library is prepared by integrating cDNA in the cloning vector as mentioned above and introducing the cloning vector into a host cell. When the cloning vector is to be a plasmid, the introduction into the host cell can be attained by electropolation or by calcium chloride method. When the cloning vector is a phage, the introduction into the host cell can be attained by in vitro packaging, etc.

A transformant containing a DNA coding for VH and VL of a mouse anti-posphocholine antibody can be obtained from the cDNA libvrary prepared above by producing a probe based on the nucleic acid sequence of a DNA coding for VH and VL of a mouse anti-phosphocholine antibody disclosed in the literature [Proc. Natl. Acad. Sci. USA, 73, 2109 (1985)], labeling the probe with a fluorescent substance, a radiation, or an enzyme, etc., and selecting a transformant capable of hybridizing with the probe by plaque hybridization, colony hybridization, Southern hybridization, or the like.

As the probe, the DNAs which have wholly or partially the nucleic acid sequence of VH shown in SEQ. ID. No. 1 and the nucleic acid sequence of VL shown in SEQ. ID. No. 2 may be mentioned.

The DNAwhich codes for VH can be obtained by amplification by Polymerase Chain Reaction (hereinafter indicated as "PCR") using a synthetic DNA having a nucleic acid sequence shown in SEQ. ID. No. 3 as a sense primer and a synthetic DNA having a nucleic acid sequence shown in SEQ. ID. No. 4 as an anti-sense primer while using a mouse spleen cell cDNA library as a template. Similarly, the DNA which codes for VL can be obtained by amplification by PCR using a synthetic DNA having a nucleic acid sequence shown in SEQ. ID. No. 5 as a sense primer and a synthetic DNA having a nucleic acid sequence shown in SEQ. ID. No. 6 as an anti-sense primer while using a mouse spleen cell cDNA library as a template. Besides, the DNA which contains the whole sequence of VH and VL by utilizing PCR.

By determining the full length of the nucleic acid sequence of a DNA coding for VH and VL of a mouse anti-phosphocholine antibody as obtained above, it is possible to estimate the full length of the amino acid sequences of VH and VL based on the determined nucleic acid sequences. (3) Construction of a human chimeric antibody expression vector

An expression vector can be constructed by inserting a cDNA coding for VH and VL of a mouse anti-phosphocholine antibody into the upstream of a gene coding for CH and CL of the antibodies of the vector for expressing an antibody constructed as described above. For example, an expression vector can be constructed by providing a recognition sequence of restriction enzyme for cloning a cDNA coding for VH and vL of a mouse anti-phosphocholine antibody in the upstream a gene coding for CH and CL of an antibody of an expression vector in advance, and inserting into the cloning site a cDNA coding for VH and VL of a mouse anti-phosphocholine antibody via of a synthetic DNA which will be specifically described herein below. The synthetic DNA comprises a nucleic acid sequence on the 3'terminal end of the V region of a mouse anti-phosphocholine antibody and a nucleic acid sequence on the 5'terminal end of the C region of an antibody on the expression vector, and the synthesized DNA can be prepared to have sites suitable for restriction enzymes at both the terminals thereof by using a DNA synthesizer.

### (4) Expression of humanized antibody

A transformant capable of stably producing an anti-phosphocholine antibody can be obtained by introducing the expression vector described above into a suitable host cell. As a means to introduce the expression vector into the host cell, the method of electropolation [JP-A-02-257891 and Cytochnology, 3, 133 (1990)] may be mentioned. As the host cell to be used for introducing the expression vector, any host cell can be used so long as an antibody is expressed therein, and the examples include mouse SP2/0-Ag cell (ATCC CRL1581), mouse P3X63-Ag8.653 cell (ATCC CRL1580), CHO cell lacking in a dihydrofolate reductase gene (hereinafter indicated as "DHFR gene") [Proc. Natl. Acad. Sci., 77, 4216 (1980)], and rat YB2/3HL.P2.G11.16Ag.20 cell (ATCC CRL1662, hereinafter referred to as "YB2/0 cell").

After the introduction of a vector, the transformant which stably produces an antibody may be selected using RPMI 1640 culture medium containing G418 and FCS in accordance with the method disclosed in JP-A-02-257891. By culturing the transformant thus obtained in a culture medium, it is possible to produce and accumulate a recombinant antibody in a culture medium. Further, the amount of the recombinant antibody can be increased by using a DHFR gene amplifying system, for example, in accordance with the method disclosed in JP-A-02-257891.

The antibody can be purified from the supernatant of the culture medium of the transformant by using a protein A column (Antibodies, Chapter 8). The antibody can also be purified by a conventional method which has been used for purified a protein can be used. For example, the purification can be effected by combining gel filtration, ion-exchange chromatography, and ultrafiltration. The molecular weight of the H chain and the L chain of the purified recombinant antibody or the molecular weight of the whole antibodymolecule may be determined by polyacrylamide electrophoresis (SDS-PAGE) [Nature, 227, 680 (1970)] and Western blotting (Antibodies, Chapter 12), etc.

As the antibody-producing cells which produce an antibody used in the method of the present invention, antibody-producing cell KTM-2001 may be mentioned. The antibody-producing cells KTM-2001 was deposited with International Patent Organism Depositary of National Institute, Advanced Industrial Science and Technology, located at AIST Tsukuba Central 6, 1-1-1 Higashi, Tsukuba, Ibaraki, Japan (Postal No. 305-8566) on April 18, 2001 under Accession No. FERM BP-7549. The antibody which is produced by the antibody-producing cell KTM-2001 will be hereinafter simply referred to as "KTM-2001 antibody".

### Brief Description of the Drawings

Fig. 1 shows the structure of a recombinant plasmid pBSPCVH.
Fig. 2 shows the structure of a recombinant plasmid pBCPCVL.
Fig. 3 shows the structure of an expression vector pKANTEX 93.
Fig. 4 shows the structure of a plasmid pBSVH-2.
Fig. 5 shows the structure of a plasmid pBSVL-2.
Fig. 6 shows the structure of a plasmid pKANTEXPCVH.
Fig. 7 shows the structure of a plasmid pKANTEXPChCγ1.
Fig. 8 shows the results of the test in which plasmas of healthy persons and patients of coronary arteriosclerosis and a sample dilution as control are diluted to 5 serial dilution. The mark -●- denotes the results of the plasma of a patient of coronary arteriosclerosis, the mark -▲- denotes the results of the plasma of a healthy person, and the mark -○- denotes the results of the sample dilution.
Fig. 9 shows the values obtained by the determination of denatured lipoprotein in the plasmas of healthy persons and patients of coronary arterial stenosis.
Fig. 10 shows the values obtained by the determination of denatured lipoprotein in the plasmas of healthy persons and patients of angina pectoris.
Fig. 11 shows the values obtained by the determination of denatured lipoprotein in the plasmas of healthy persons and patients of myocardial infarction.
Fig. 12 shows the determination results of lipoproteins derived from plasmas of healthy persons and patients of coronary arteriosclerosis, in case of subjecting the lipoproteins in advance to various physical degenerations. The mark -○- denotes the results of the plasma of healthy person and the mark -●- denotes the results of the plasma of patient.
Fig. 13 shows the determination results of lipoproteins of healthy persons, in case of subjecting the lipoproteins in advance to enzymatic degenerations caused by the action of lipoprotein lipase.
Fig. 14 shows the determination results of the reactivity of purified lipoprotein with KTM-285 antibody and KTM-2001 antibody, in case of subjecting the purified lipoprotein in advance to degeneration.
Fig. 15 shows the determination results of the reactivity of purified lipoprotein with KTM-285 antibody, in case of subjecting the lipoprotein in advance to degeneration.

Now, the present invention will be specifically described below with reference to examples. The present invention is not restricted to the examples.

### Best Mode for Carrying out the Invention

### Example 1: Production of anti-phosphocholine antibody (KTM-2001 antibody)

### (1) Preparation of mouse anti-phosphocholine antibody gene 1) Construction of cDNA library from mouse spleen cell

A Balb/c mouse which was bred ordinarily was sacrificed by removal of whole blood and subjected to peritoneotomy to excise the spleen. The spleen was minced in a RPMI 1640 culture medium (produced by Nissui Seiyaku K..), loosened with forceps, and centrifuged (1200 r.p.m. for five minutes). A supernatant was discarded, and the residue was treated with a Tris-ammonium chloride buffer (pH 7.65) for one to two minutes to remove erythrocytes, and washed three times with a RPMI1640 culture medium, and further washed three times with physiological saline, and used for the extraction of mRNA. The mRNA was obtained from 5.0 x 10⁷ of the spleen cells by the use of an mRNA extraction kit, Quick Prep. mRNA purification kit (product code: 27-9254-01, Amasham-Pharmacia Corp.) according to the manual attached to the kit.

A cDNA having Eco RI-Not I adapters at the opposite ends thereof was synthesized from 5 µg of the mRNA by using a cDNA Synthesis Kit (product code: 27-9260-01, Pharmacia Corp.) in accordance with the manual attached to the kit. About 6 µg of each cDNA thus obtained was dissolved in 10 µg of sterilized water and fractionated by agarose gel electrophoresis to recover about 1.8 kbp of a cDNA fragment corresponding to the H chain of an antibody and about 1.0 kbp of a cDNA fragment corresponding to the L chain each in an amount of about 0.1 µL. Then, 0.1 µL of about 1.8 kbp of cDNA fragment corresponding to the H chain and 0.1 µL of about 1.0 kbp of cDNA fragment corresponding to the L chain and 1 µL of Lambda ZAPII vector [obtained by cleaving a Lambda ZAPII vector with an Eco RI and then treated with a calf intestine alkaline phosphatase: manufactured by Stratagene Corp.] were dissolved in 11.5 µL of T4 ligase buffer. Then, 175 units of T4 DNA ligase was added, and the resultant mixture was incubated at 12°C for 24 hours, and further incubated at room temperature for two hours. 4 µL of each of the reaction solutions was packaged in a lambda phage by using a Giga Pack Gold (product code: SC200201, Stratagene Corp.) according to a conventional method [Molecular Cloning, 2, 95, compiled by Maniatis et al. and published by Cold Spring Harbor Laboratory in 1989]. The packaged reaction solutions were infected to Escherichia coli strain XLI-Blue MRF' annexed to the Giga Pack Gold [Biotechniques, 5, 376 (1987)] according to a conventional method [Molecular Cloning, 2, 95-107, compiled by Maniatis et al. and published by Cold Spring Harbor Laboratory in 1989] to obtain a phage clone as a mouse spleen cell cDNA library. Then, the phage was fixed on a HyBand N⁺ filter (product code: RPN87B, Amasham-Pharmacia Corp.) according to a conventional method [Molecular Cloning, 2, 112, compiled by Maniatis et al. and published by Cold Spring Harbor Laboratory in 1989].

### 2) Preparation of probe DNA for VH

In an Ex Taq. polymerase reaction solution (manufactured by Takara Shuzo, Co., Ltd.) containing each 12.5 pmoles of primers having nucleic acid sequences of SEQ. ID. No. 7 and SEQ. ID. No. 8, 10 ng of a mouse spleen cell cDNA obtained in 1) above and 200 µmol/L deoxynucleotide triphosphoric acid, one unit of Ex Taq. polymerase (product code: RR001A, Takara Shuzo, Co., Ltd.) was added, pretreated at 95°C for five minutes, and subjected to polymerase chain reaction (PCR) at 95°C for two minutes, at 55°C for two minutes, and at 72°C for two minutes for 30 cycles, to recover about 260 bp of a DNA fragment.

All the PCR reactions were carried out by using a GeneAmPCR System 9700 (manufactured by Perkin Elmer Corp.). The sequence of the amplified fragment was determinde by the method shown in 5 below, and the sequence was confirmed to be consistent with the nucleic acid sequence of a mouse anti-phosphocholine antibody H chain cDNA (GENBANK Accession No. M16334).

### 3) Preparation of probe DNA for VL

In an Ex Taq. polymerase reaction solution (manufactured by Takara Shuzo, Co., Ltd.) containing each 12.5 pmoles of primers having nucleic acid sequences of SEQ. ID. No. 9 and SEQ. ID. No. 10, 1.0 ng of a mouse spleen cell cDNA produced in 1) above, and 200 µmol/L deoxynucleotide triphosphoric acid, one unit of Ex Taq. polymerase (product code: RR001A, Takara Shuzo, Co., Ltd.) was added, pretreated at 95°C for five minutes, and subjected to PCR reaction at 95°C for two minutes, at 55°C for two minutes, and at 72°C for two minutes for 30 cycles, to recover about 220 bp of a DNA fragment. The sequence of the amplified fragment was determined by the method shown in 5 below, and the sequence was confirmed to be consistent with the nucleic acid sequence of a mouse anti-phosphocholine antibody L chain cDNA (GENBANK Accession No. U29423).

### 4) Cloning of mouse anti-phosphocholine antibody cDNA

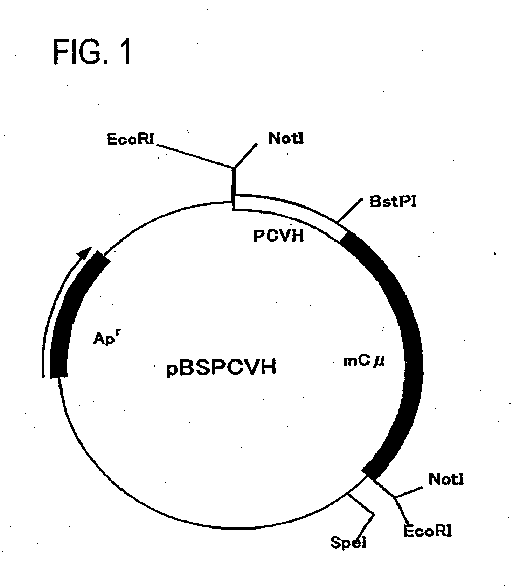

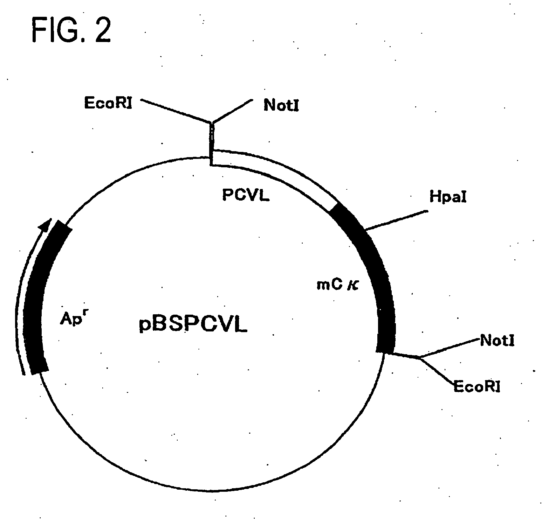

From a membrane having transcribed thereto the mouse spleen cell cDNA library produced in 1) above, the phage clone strongly bound to the probe produced in 2) and 3) above and labeled in accordance with the protocol annexed to the ECL Direct Labeling and Detection Kit (product code: RPN3000, Amasham-Pharmacia Corp.) was selected in accordance with a conventional method [Molecular Cloning, 2, 108, compiled by Maniatis et al. and published by Cold Spring Harbor Laboratory in 1989]. Then, the phage clone was sub-cloned to a plasmid pBluescript SK(-) by using a cDNA synthesis kit, a ZAP-cDNA Synthesis Kit (product code: SC200400, Stratagene Corp.) to obtain a recombinant plasmid pBSPCVH containing an H chain cDNA of a mouse anti-phosphocholine antibody (Fig. 1) and a recombinant plasmid pHSPCVL containing an L chain cDNA of a mouse anti-phosphocholine antibody (Fig. 2). The pBSPCVH and the pBSPCVL were respectively cleaved with Eco RI, and it was found that about 1.8 kbp of a cDNA fragment was inserted in pBSPCVH, and about 1.1 kbp of the cDNA fragment was inserted in pBSPCVL.

### (2) Nucleic acid sequence of variable regions of H chain cDNA and L chain cDNA

The nucleic acid sequences of the variable regions of the H chain cDNA and the L chain cDNA obtained in 4) above were determined in accordance with a Dye Deoxy method [Molecular Cloning, 13.42, compiled by Maniatis et al. and published by Cold Spring Harbor Laboratory in 1989] using the Dye Terminator Cycle Sequencing FS Ready Reaction Kit (product: 402123, Perkin Elmer Corp.). Both sequences included methionine, which is assumed to be an initiating codon, ATG, at the 5'-terminal, and were cDNAs of a full length of cDNAs including a leader sequence. The nucleic acid sequence of VH is shown in SEQ. ID. No. 1 and the nucleic acid sequence of VL is shown in SEQ. ID. No. 2. (3) Construction of recombinant antibodyexpressing vector 1) Construction of human IgG1 type recombinant antibodyexpressing vector

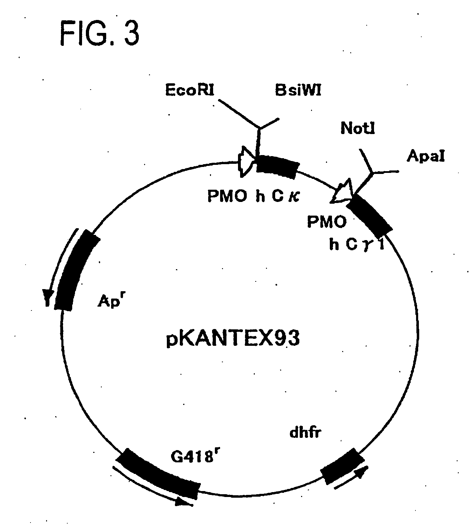

The variable regions of the H chain cDNA and the L chain cDNA obtained in 4) above were inserted to a human IgG1 type chimeric antibodyexpressing vector pKANTEX93 (Fig. 3) described in WO 97/10354 in accordance with the procedure described below.

### 2) Insertion of VH region of mouse anti-phosphocholine antibody

For the purpose of fusing cDNA of a mouse VH region with a human constant region by genetic engineering, two kinds of synthetic DNA represented by SEQ. ID. No. 3 and SEQ. ID. No. 4 each in an amount of 25 pmole were dissolved in 10 µL of a buffer containing 10 mmol/L Tris-HCl (pH 7.5), 10 mmol/L magnesium chloride, 1 mmol/L DTT, and 50 mmol/L sodium chloride, reacted in a water bath at 70°C for 10 minutes, and gradually cooled to effect association reaction to produce a double stranded synthetic DNA cassette.

Meanwhile, 1 µg of plasmid pBSPCVH containing a full length of cDNA of H chain was dissolved in 30 µL of a buffer containing 10 mmol/L Tris-HCl (pH 7.5), 10 mmol/L magnesium chloride, 1 mmol/L DTT, and 50 mmol/L sodium chloride, mixed with 10 units of BstPI and 10 units of SpeI, and digested at 37°C for two hours. The resultant reaction mixture was subjected to agarose gel electrophoresis to recover about 3.4 kbp of a DNA fragment containing the VH region of cDNA and Ap-resistant gene.

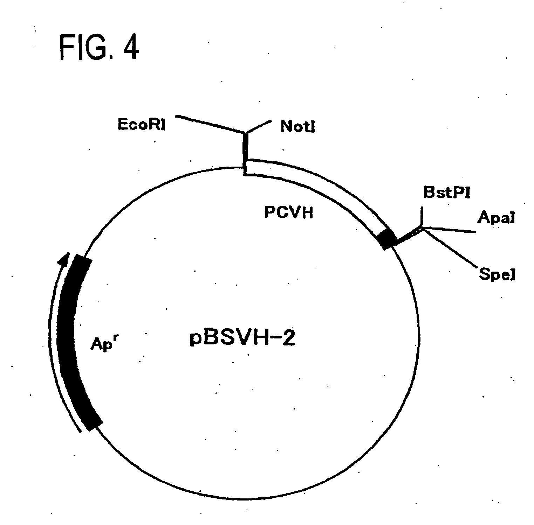

0.1 µg of the BstPI-SpeI fragment (3.4 kbp) derived from the plasmic pBSPCVH and the double stranded synthetic DNA cassette (10 fmole) obtained above were dissolved in 9 µL of sterilized water, mixed with 9 µL of a DNA ligation kit ver. 2 Solution I (product code: 6022, Takara Shuzo, Co., Ltd.), and ligated at 16°C for one hour. Escherichia coli DH5α strain was transformed with a recombinant plasmid DNA thus obtained and the plasmid pBSVH-2 shown in Fig. 4 was obtained

Then, 1 µg of the pBSVH-2 obtained above was dissolved in 30 µL of a buffer containing 10 mmol/L (pH 7.5), 10 mmol/L magnesium chloride, and 1 mmol/L DTT, mixed with 10 units of ApaI, and digested at 37°C for two hours. To the reaction mixture was added a 60 pL of buffer containing 50 mmol/L Tris-HCl (pH 7.5), 10 mmol/L magnesium chloride, 1 mmol/L DTT, 100 mmol/L sodium chloride, 0.01% Triton X-100 and 0.01% BSA, and mixed with 10 units of NotI, and digested at 37°C for two hours. The resulting reaction mixture was subjected to agarose gel electrophoresis to recover about 0.5 kbp of DNA fragment containing CDNA of the VH region.

Meanwhile, 1 µg of the human type chimeric antibodyexpression vector pKANTEX93 shown in Fig. 3 was dissolved in 30 µL of a buffer containing 10 mmol/L Tris-HCl (pH 7.5), 10 mmol/L magnesium chloride, and 1 mmol/L DTT, mixed with 10 units of ApaI, and digested at 37°C for two hours. To the reaction mixture was added a 60 µL of buffer containing 50 mmol/L Tris-HCl (pH 7.5), 10 mmol/L magnesium chloride, 1 mmol/L DTT, 100 mmol/L sodium chloride, 0.01% Triton X-100 and 0.01% BSA, and mixed with 10 units of NotI, and digested at 37°C for two hours. The resulting reaction mixture was subjected to agarose gel electrophoresis to recover about 13.2 kbp of a DNA fragment containing cDNA of human γ1H chain constant region, cDNA of human κL chain constant region, two promoters of mouse Moloney leukemia virus, two splicing signals, a dehydrofolate reductase gene and G418 resistant gene.

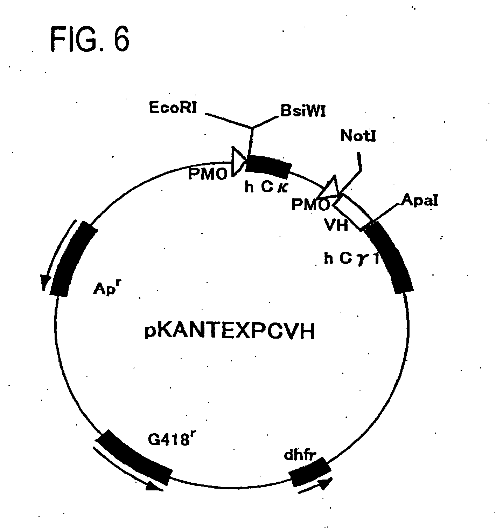

0.05 µg of the NotI-ApaI fragment (0.5 kbp) derived from the plasmid pBSVH-2 (Fig. 4) obtained above and 0.1 µg of the NotI-ApaI fragment (13.2 kbp) derived from the pKANTEX93 were dissolved in 9 µL of sterilized water, mixed with 9 µL of the DNA ligation kit ver. 2 Solution I (manufactured by Takara Shuzo, Co., Ltd.), and ligated at 16°C for one hour. Escherichia coli DH5α strain was transformed with the recombinant plasmid DNA thus obtained to obtain a plasmid pKANTEXPCVH shown in Fig. 6.

### 3) Insertion of mouse anti-phosphocholine antibody VL region

For the purpose of fusing cDNA of a mouse VH region with a human constant region by genetic engineering, two kinds of synthetic DNA represented by SEQ. ID. No. 5 and SEQ. ID. No. 6 each in an amount of 25 pmole were dissolved in 10 µL of a buffer containing 10 mmol/L Tris-HCl (pH 7.5), 10 mmol/L magnesium chloride, 1 mmol/LDTT, and 50 mmol/L sodium chloride, reacted in a water bath at 70°C for 10 minutes, and gradually cooled to effect association reaction to produce a double stranded synthetic DNA cassette.

Meanwhile, 1 µg of plasmid pBSPCVL containing a full length of cDNA of L chain was dissolved in 30 µL of a buffer containing 50 mmol/L Tris-HCl (pH 7.5), 10 mmol/L magnesium chloride, 1 mmol/L DTT, and 100 mmol/L sodium chloride, mixed with 10 units of EcoRI and 10 units of SfcI, and digested at 37°C for two hours. The resultant reaction mixture was subjected to agarose gel electrophoresis to recover about 0.4 kbp of a DNA fragment containing the VL region of cDNA.

Further, 1 µg of a cloning vector pBluescript SK(-) (manufactured by Stratagene Corp) was dissolved in 30 µL of a buffer containing 20 mmol/L Tris-HCl (pH 8.5), 10 mmol/L magnesium chloride, 1 mmol/L DTT, and 100 mmol/L potassium chloride, mixed with 10 units of EcoRI and 10 units of BamHI, and digested at 37°C for two hours. The resultant reaction mixture was subjected to agarose gel electrophoresis to recover about 2. 9kbp of a DNA fragment containing the cloning vector pBluescript SK(-).

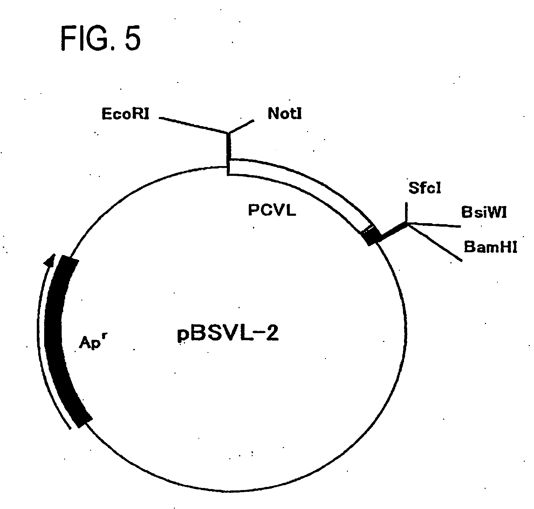

0.1 µg of the EcoRI-SfcI fragment (0.4 kbp) derived from the plasmid pBSPCVL, 0.1 µg of the Eco RI-Bam HI fragment (2.9 kkbp) derived from the plasmid pBluescroipt SK (-), and the double stranded synthetic DNA cassette (10 fmole), which were obtained above, were dissolved in 9 µL of sterilized water, mixed with 9 µL of a DNA ligation kit ver. 2 Solution I (product code: 6022, Takara Shuzo, Co., Ltd.), and ligated at 16°C for one hour. Escherichia coli DH5α strain was transformed with the recombinant plasmid DNA thus obtained, and the plasmid pBSVL-2 shown in Fig. 5 was obtained. Then, 1 µg of the pBSVL-2 thus obtained was dissolved in 30 µL of a buffer containing 50 mmol/L Tris-HCl (pH 7.5), 10 mmol/L magnesium chloride, 1 mmol/L DTT, and 100 mmol/L sodium chloride, mixed with 10 units of EcoRI and 10 units of BsiWI, and digested at 37°C for two hours. The resultant reaction mixture was subjected to agarose gel electrophoresis to recover about 3.4 kbp of a DNA fragment containing cDNA of the VL region.

Meanwhile, 1 µg of the expressing plasmid pKANTEXPCVH obtained in 2) of (3) above was dissolved in a buffer containing 50 mmol/L Tris-HCl (pH 7.5), 10 mmol/L magnesium chloride, 1 mmol/L DTT, and 100 mmol/L sodium chloride, mixed with 10 units of EcoRI and 10 units of BsiWI, and digested at 37°C for two hours. The resultant reaction mixture was subjected to agarose gel electrophoresis to recover about 13.7 kbp of a DNA fragment containing cDNA of mouse anti-phosphocholine antibody H-chain variable region, cDNA of human γ1H chain constant region, cDNA of human κL chain constant region, two promoters of mouse Moloney leukemia virus, two splicing signals, a dehydrofolate reductase gene, and G418 resistant gene.

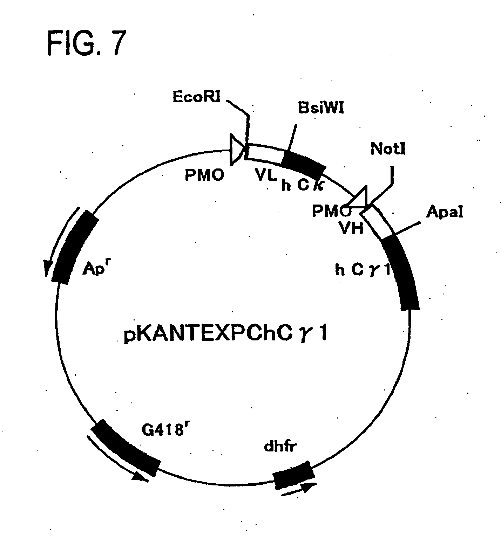

0.05 µg of the EcoI-BsiWI fragment (0.5 kbp) derived from the plasmid pBSVL and 0.1 µg of the EcoI-BsiWI fragment (13.7 kbp) derived from the pKANTEXPCVH, which were obtained above, were dissolved in 9 µL of sterilized water, mixed with 9 µL of the DNA ligation kit ver. 2 Solution I (manufactured by Takara Shuzo, Co., Ltd.), and ligated at 16°C for one hour. Escherichia coli DH5α strain was transformed with the recombinant plasmid DNA thus obtained to obtain a plasmid pKANTEXPChCγ1 shown in Fig. 7.

### (4) Expression of recombinant anti-phosphocholine antibodyexpressing vector

The introduction of mouse chimera anti-phosphocholine antibody-expressing vector into YB2/0 cells was carried out by electropolation in accordance with the method proposed by Miyaji et al. [Cytotechnology, 3, 133 (1990)]. After the' introduction of 4 µg of chimera anti-phosphocholine antibody expression vector into 4 x 10⁶ of YB2/0 cellls (ATCC CEL1581), the cells were suspended in 40 ml of RPMI 1640-FCS (10) [RPMI 1640 culture medium containing 10% of FCS (manufactured by Gibco)]. The resultant suspension was dispensed each in 96-well microtiter plate in an amount of 200 µL, incubated in a CO₂ incubator at 37°C for 24 hours. Then, G418 (manufactured by Gibco) was added thereto to the final concentration of 0.5 mg/ml, and the mixture was cultured for one'to two weeks. From the wells in which colonies of transformant were formed and became confluent, the culture medium was recovered and subjected to enzyme immunoassay (ELISA method) shown below to determine the amount of the recombinant antibody expressed.

### (5) Method of enzyme immunoassay (ELISA method)

### 1) ELISA for determination of antibody activity

The PC9CHO-LDL conjugate prepared in (2) of Example 2 was adjusted to 1 µg/mL with PBS. 50 µL of this solution or 50 µL of the diluted solution was dispensed to 96-well microtiter plate (manufactured by Falcon Corp), air-dried, and blocked with PBS containing 0.1% BSA. The blocked conjugate was mixed with a supernatant of the culture medium of the transformant or with the purified recombinant anti-phosphocholine antibody in an amount of 50 to 100 µL, and reacted at room temperature for one to two hours. After the reaction, each of the wells was washed with PBS, mixed with 50 to 100 µL of the peroxidase-labeled anti-human γ1 antibody (product code: A110POD, American Calex Corp.), and reacted at room temperature for one to two hours. After the reaction products in the wells were washed with PBS, they were subjected to color development by the addition of 50 to 100 µL of the ABTS substrate solution [a solution obtained by dissolving 550 mg of 2,2'-azinobis(3-ethylbenzothiazolin-6-sulfornic acid) diammonium in 0.1 mol/L of citrate buffer solution (pH 4.2), and mixed with 1 µL /mL of hydrogen peroxide just before use] to determine the absorbance at 415 nm.

### 2) ELISA for determination of amount of antibody

50 µL of a diluted solution of anti-human κL-chain antibody (product code: 05-3900, Zymed Corp.) was dispensed to the 96-well microtiter plate (manufactured by Falcon Corp), air-dried, and blocked with PBS containing 0.1% BSA. The blocked solution in the wells was mixed with a supernatant of the culture medium containing the transformant or with the purified recombinant anti-phosphocholine antibody in an amount of 50 to 100 µL, and reacted at room temperature for one to two hours. After the reaction, each of the wells was washed with PBS, mixed with 50 to 100 µL of the peroxidase-labeled anti-human γ1 antibody (product code: A110POD, American Calex Corp.), and reacted at room temperature for one to two hours. After the reaction products in the wells were washed with PBS, they were subjected to color development by the addition of 50 to 100 µL of the ABTS substrate solution [a solution obtained by dissolving 550 mg of 2,2'-azinobis(3-ethylbenzothiazolin-6-sulfornic acid) diammonium in 0.1 mol/L of citrate buffer solution (pH 4.2), and mixed with 1 µL /mL of hydrogen peroxide just before use] to determine the absorbance at 415 nm.

### (6) Preparation of highly expressing strain by gene amplification

The clone obtained above was suspended in the RPMI 1640-FCS (10) culture medium containing 0.5 mg/mL of G418 and 50 nmol/L of methotrexate (hereinafter abbreviated as "MTX") at a density of 1.0 to 2.0 x 10⁵ cells/mL. The resultant suspension was dispensed to each a 24-well plate in an amount of 1 mL/well. The suspensions in the wells were incubated in a CO₂ incubator at 37°C for one to two weeks to induce a clone resistant to 50 nmol/L MTX. The 50 nmol/L MTX-resistant clone thus obtained was suspended in the RPMI 1640-FCS (10) culture medium containing 0.5 mg/mL G418 and 100 nmol/L MTX at a density of 1.0 to 2.0 x 10⁵ cells/mL and the resultant suspension was dispensed to a 24-well plate in an amount of 1 mL/well, incubated in a CO₂ incubator at 37°C for one to two weeks to induce a clone resistant to 100 nmol/L MTX. The 100 nmol/L MTX-resistant clone thus obtained was suspended in the RPMI 1640-FCS (10) culture medium containing 0.5 mg/ml G418 and 200 nmol/L MTX at a density of 1.0 to 2.0 x 10⁵ cells/mL and the resultant suspension was dispensed to a 24-well plate in an amount of 1 mL/well.

The resultant suspension was incubated in a CO₂ incubator at 37°C for one to two weeks to induce a clone resistant to 200 nmol/L MTX. When the clones in the wells became confluent, they were subjected to ELISA method to determine the amounts of chimera antibodies expressed. Among the clones thus obtained, the 200 nmol/L MTX resistant clone which showed the highest amount of expression was subjected to single cell cloning by limiting dilution. When the clone became confluent, it was subjected to ELISA method to determine the amounts of chimera antibodies expressed. Among the clones thus obtained, the 200 nmol/L MTX-resistant clone that showed the highest amount of expression was produced about 10 µg/mL of chimeric antibody. The 200 nmol/L MTX-resistant clone obtained from the YB2/0 cells having pKANTEXPChCγ1 introduced was called as the transformant KTM-2001 (human type chimeric antibody KTM-2001-producing strain).

### Example 2: Preparation of anti-phosphocholine antibody (KTM-285 antibody)

### (1) Preparation of LDL fraction in human plasma

Human plasma obtained from heparinized blood was mixed with EDTA so as to give a final concentration of 0.25 mmol/L. The resultant mixture was put to test tubes for supercentrifugation (4 mL in volume) each in an amount of 0.75 mL. Then, 250 µL of 0.15 mol/L NaCl containing 0.3 mmol/L EDTA was superposed thereon, and centrifuged at 185,000 x g at 10°C for 2.5 hours. While 150 µL of the upper layer was discarded, 750 µL of the lower layer was collected and mixed with 150 µL of a KBr solution (50 w/v%) to give a density of 1.063. The plasma having the density adjusted was transferred to the bottom of the test tube for supercentrifugation (4 mL in volume), centrifuged at 240, 000 x g at 10°C for 16 hours. The orange band (about 100 to 150 µL) in the upper layer was carefully recovered and dialyzed against PBS containing 0.25 mmol/L EDTA at 4°C for six hours (3 liters was replaced three times at an interval of two hours). The LDL sample thus obtained had the LDL purity thereof confirmed by showing a single band in agarose electrophoresis using lipid staining methanol and then tested by Lowry method for the protein content with BSA as the standards. This value thus found was reported as the LDL concentration.

### (2) Preparation of antigen for immunization and antigen for solid phase

1-Palmitoyl-2-(9-oxononanoyl)-glycelo-3-phosphochol ine was prepared in accordance with the method disclosed in the literature [J. Biol. Chem., 266(17), 11095 (1991)]. Specifically, ozone gas was blown into a chloroform solution of 1-palmitoyl-2-oleoil 10-glycero-3-phosphocholine (manufactured by Avanti Corp) and the formed ozonide body of diacylglycerophosphocholine was reduced with dimethyl sulfide, and the resultant oxide was developed by thin film chromatography in a developing solvent (chloroform : methanol : water = 10 : 5 : 1) to isolate 1-palmitoyl-2-(9-oxononanoyl)-glycero-3-phosphocholine (hereinafter abbreviated as "PC9CHO"). The purity of PC9CHO was confirmed to show a single peak by the reversed phase HPLC (ODS column, methanol : aqueous 20 mmol/L choline chloride solution : acetonitrile = 875 : 100 : 25), and was preserved in chloroform/ethanol (1 : 1) solution at -20°C.

The purified LDL obtained in the paragraph (1) above was dissolved in PBS solution containing 0.25 mmol/L EDTA so as to give a concentration of 1 mg/mL. To 1 mL of this solution was added 100 µL of a DMSO solution having dissolved 5, 000 ng of the PC9CHO mentioned above to obtain the PC9CHO-LDL conjugate.

### (3) Preparation of monoclonal antibody

Male Balb/c mice of six weeks old were subcutaneously administered to the back thereof the phosphocholine LDL which was mixed with the same amount of Freund's complete adjuvant at a dose of 0.1 mg/mouse. Thereafter, 0.1 mg/mouse of the mixture at equivalence was subcutaneously administered to the back of mice twice at intervals of three weeks. After three weeks, the PC9CHO-LDL conjugate dissolved in PBS was administered to the mice through caudal vein at a dose of 0.1 mg/mouse. After three days , the antibody-producing cells were taken from the spleen as shown below.

The spleens were aseptically extracted from the immunized animals, dissolved in a RPMI-1640 culture medium (manufactured by Nissui Seiyaku K.K.) containing no serum, separated into individual cells by being passed through a net of 100 meshes, suspended in a hypotonic solution to dissolve erythrocytes, and then washed by centrifugation three times with the RPMI-1640 culture medium containing no serum to obtain antibody-producing cells.

Meanwhile, the mouse myeloma cells P3X-Ag8-UI were cultured in a RPMI-1640 culture medium containing 10% fetal calf serum (FCS). The cells were recovered from the culture broth in the logarithmic growth phase and washed by centrifugation three times with the RPMI-1640 culture medium containing no serum.

The antibody-producing cell suspension and the mouse myeloma cell P3X-Ag8UI suspension obtained as described above were mixed at a ratio of 10 : 1 and centrifuged at 1200 rpm for five minutes to remove the culture medium. To the remaining cells was added slowly 1 mL of the 50% polyethylene glycol 1500 solution (manufactured by Broeringer-Mannheim Corp), and 50 mL of the RPMI-1640 culture medium containing no serum was further added thereto slowly, and centrifuged at 1200 rpm for five minutes to remove the culture medium. The residual cells were suspended in a HAT culture medium (RPMI-1640 culture medium containing 10% FCS and also containing 1 x 10⁻⁴ mol/L hypoxanthine, 4 x 10⁻⁷ mol/L aminopterin, and 2 x 10⁻⁵ mol/L thymidine) so as to give a density of 1 x 10⁶ cells/mL. The resultant suspension was dispensed into a 96-well microtiter plate in an amount of 200 µL/well. The cells in intact form were cultured in air containing 5% CO₂ at 37°C. After 10 days, a colony of hybridoma was observed in all the wells.

In order to select wells containing cells producing a target antibody, the supernatants of the culture medium were subjected to ELISA to determine the antibody titer. 50 µL of the PC9CHO-LBL conjugate solution (20 µg/mL in 0.1 mol/L carbonate buffer, pH 9.5) or the LDL solution (control, 20 µg/mL in 0.1 mol/L carbonate buffer, pH 9.5) was dispensed to the 96-well microtiter plate and allowed to stand at 4°C overnight. The plate was washed three times with the PBS. Then, 250 µL of the 1%BSA/PBS solution was dispensed to the wells and allowed to stand at room temperature for one hour. The wells were washed three times with PBS to prepare the plate for the reaction. In the plate for reaction plate, 50 µL of the culture supernatant diluted to 11 times the original volume with PBS containing 0.1% of BSA was placed and allowed to stand and reacting at room temperature for three hours. After the reaction, the plate was washed five times with PBS containing 0.05% Tween 20. In the washed plate, 50 µL of peroxidase (POD)-labeled anti-mouse immunoglobulin rabbit IgG (manufactured by Daco Corp) was added, and reacted at room temperature for one hour. After the reaction, the plate was washed five times with the PBS containing 0.05% Tween 20. In the washed plate, 50 µL of the TMB coloring reagent solution (manufactured by Intergene Corp) was placed and reacted at room temperature for 30 minutes, and finally mixed with 50 µL of an aqueous 1 mol/L sulfuric acid solution, and the absorbance at 450 nm was determined with a microplate reader (product cod: MTP-120, Corona Denki K.K.). The cells in the wells which showed the increase in absorbance of not less than 1.0 as compared with the control were selected.

The cloning was carried out by limiting dilution. The cells in the well obtained above were diluted with the 10% FCS-containing RPMI-1640 culture medium containing 1 x 10⁷/mL thymocytes so as to give a density of 0. 5 cell/mL. The diluted cells were dispensed to a 96-well microtiter plate in an amount of 200 µL, and cultured in a 5% CO₂ incubator at 37°C. After 10 to 14 days from the start of the cultivation, the wells were observed to select the wells containing one growing colony per well. The supernatants of the culture medium in the wells are subjected to ELISA to determine the antibody titers. The wells containing cell strains which produced the target antibody were selected. The same procedure was further carried out twice, and the monoclonal antibody-producing cell strain which stably produced the target antibody was obtained. The immune globulin class of the antibody produced by the resultant stain was identified with the antibody in the supernatant of the culture medium by using a monoclonal antoibody typing kit (manufactured by Zymed Corp) and was collected. The antibody was of the IgM type.

Male Balb/c mice of 8 weeks old or more were intraperitonealy injected with pristane (2, 6, 10, 14-tetramethyl pentadecane) at a dose of 0. 5 mL/mouse and then bred for two weeks.

The mice were intraperitonealy inoculated with monoclonal antibody-producing cells at a dose of 1 x 10⁶ cells/mouse.

After 7 to 14 days, when the mice pooled ascites sufficiently in their abdominal cavities, the ascites was taken from the abdominal cavities with a needle of 18G and centrifuged at 3, 000 rpm for 10 minutes to obtain a supernatant. The supernatant was diluted 3-fold with PBS. To the diluted ascites was dropwise added the same amount of a saturated ammonium sulfate/PBS solution with stirring. The resultant mixture was further stirred for a while after the dropwise addition was completed. The resultant mixture was centrifuged at 3, 000 rpm for 10 minutes to recover a residue. This residue was dissolved with the PBS of an amount equal in volume to the 3-fold diluted ascites and was subjected repeatedly to the same procedure as described above. The resultant residue was dissolved in PBS containing 0.5 mol/L of NaCl, the amount of PBS is same as that of the ascites. The obtained solution was passed through a Sephacryl S-300 column equilibrated in advance with the PBS containing 0.5 mol/LNaCl, and the adsorbate was eluted with the PBS containing 0.5 mol/L NaCl. The absorbance of eluate at 280nm was continuously monitored to recover the peak part in the neighborhood of a molecular weight of 800 KD. The recovered peak part was used as a purified antibody.

### (4) Selection of antibody

The amount 50 µL of the PC9CHO-LDL solution prepared in (2) above (20 µg/mL in 0.1 mol /L carbonate buffer, pH 9.5) was dispensed to a 96-well microtiter plate (manufactured by Nunc Corp) in an amount of 50µL/well, and then allowed to stand at 4°C overnight. The plate was washed three times with PBS. Then, 250 µL of 1% BSA/PBS solution was dispensed to the wells of the plate and allowed to stand at room temperature for one hour. The wells of the plate were washed three times with the PBS, and the plate was used for the purpose of the reaction. To the plate for the reaction was added 50 µL of 0.1% BSA-containing 0.1 mol/L phosphate buffer (pH 7.4) containing various substances at various concentrations or 50 µL of 0.1% BSA-containing 0.1 mol/L phosphate buffer (pH 7.4) alone (control: inhibition 0%), and 50 µL a dilution obtained by diluting the antibody obtained in (3) above to a concentration of 100 ng/mL with 0.1 mol/L phosphate buffer (pH 7.4) containing 0.1% BSA was dispensed to the wells of the plate with shaking, and the mixture was subjected to stationary reaction at 4°C overnight. After the reaction, the plate was washed five times with the PBS containing 0.05% Tween 20, and 50 µL of the POD-labeled anti-mouse immunoglobulin-rabbit IgG was placed and reacted at room temperature for one hour. After the reaction was completed, the plate was washed five times with the PBS containing 0.05% Tween 20. On the washed plate, 50 µL of the TMB coloring solution (manufactured by Intergene Corp) was added thereto, allowed to react at room temperature for 30 minutes. Finally, 50 µL of a reaction terminating liquid was added, and the reaction product was tested with a microplate reader to determine the absorbance at 450 nm. The reactivity was determined by preparing a concentration/absorbance curve of phosphocholine used in the reaction and comparing the reaction inhibiting ratios of various substances used at various concentrations, with the absorbance of the control as 100%. On the basis of the results, a strain which showed the inhibition with phosphocholine at the lowest concentration was selected from the monoclonal antibody-producing strains obtained in (3) above. This strain was denominated as KTM-285.

### Example 3: Specificity of antibody

The PC9CHO-LDL solution (20 µg/mL in 0.1 mol/L carbonate buffer, pH 9.5) prepared in Example 2(2) was dispensed to a 96-well microtiter plate (manufactured by Nunc Corp) in an amount of 50 µl/well and allowed to stand at 4°C overnight. After the plate was washed three times, 1% BSA/PBS solution was added thereto in an amount of 250 µl/well, and allowed to stand at room temperature for one hour. The wells were washed with the PBS with three times, and the plate was used for the reaction. On this reacting plate, 50 µL of the 0.1% BSA-containing 0.1 mol/L phosphate buffer (pH 7.4) containing various substances at various concentrations or the 0.1% BSA-containing 0.1 mol/L phosphate buffer (pH 7.4) alone (control: inhibition 0%) was added and 50 µL of a dilution prepared by diluting the KTM-285 antibody obtained in Example 2 and the KTM-2001 antibody obtained in Example 1 to a concentration of 100 ng/mL with the 0.1% BSA-containing 0.1 mol/L phosphate buffer (pH 7.4) was added thereto with shaking and the mixture was allowed react at 4°C overnight. After the reaction, the plate was washed five times with the PBS containing the 0.05% Tween 20, and 50 µL of the POD labeled anti-mouse immunoblogulins rabbit IgG was added thereto and allowed to react at room temperature for one hour. After the reaction, the plate was washed five times with the PBS containing 0.05% Tween 20, and 50 µL of a TMB coloring solution (manufactured by Intergene Corp) added thereto and reacted at room temperature for 30 minutes. Finally, 50 µL of a reaction terminating liquid was added and the reaction product was tested with a microplate reader to determine the absorbance at 450 nm. The reactivity was determined by preparing a concentration/absorbance curve of phosphocholine used in the reaction and comparing the reaction inhibiting ratios of various substances at various concentrations, with the absorbance of the control as 100%. The KTM-285 antibody and the KTM-2001 antibody were both observed to be inhibited only by phosphocholine at low concentrations, and induced substantially no inhibition by any of phosphoserine, phosphothreonine, nitrophenyl phosphocholine, phosphatidylcholine, phosphoryl ethanol amine, purified human LDL and purified human HDL.

### Example 4: Determination of denatured lipoprotein in vivo by use of KTM-2001 antibody and correlation with disease (1) Preparation of peroxidase labeled antibody

To 1 mL of a purified anti-human Apo B antibody (goat, manufactured by Kappel Corp) solution (5 mg/mL, 0.1 mol/L borate buffer, pH 8.0), 50 µL of 2-iminothiolan-HCl solution (60 mmol/L, 0.1 mol/L borate buffer, pH 8.0) was added, and reacted at 30°C for 30 minutes. The resultant mixture was passed through a Sephadex G-25 column (1 cm x 30 cm, manufactured by Amersham-Pharmacia Corp) equilibrated in advance with 0.1 mol/L phosphate buffer (pH 6.0) containing 5 mmol/L EDTA to recover the eluted antibody fraction. To 1 mL of horse radish peroxidase (abbreviated as "HRP," manufactured by Toyobo Co., Ltd.) Solution (10 mg/mL in 0.1 mol/L phosphate buffer, pH 7.0), 50 µL of the EMCS solution (50 mmol/L, DMSO solution, manufactured by Doj in Kagaku K.K.) was added and reacted at 30°C for 30 minutes. The reaction mixture was passed through the Sephadex G-25 column (1 cm x 30 cm, Amersham-Pharmacia Corp) equilibrated in advance with 0.1 mol/L phosphate buffer (pH 6.5) to recover the eluted HRP fraction. The antibody fraction and the HRP fraction recovered above were mixed, allowed to react at 30°C for 30 minutes, and then passed through the Sephadex G-200 column (1 cm x 100 cm, manufactured by Amersham-Pharmacia Corp) equilibrated in advance with 0.1 mol/L phosphate buffer (pH 7.0) to recover the eluted antibody-HRP conjugate fraction, which was used as peroxidase labeled anti-human Apo B antibody. To the recovered fraction was immediately added the BSA so as to give a final concentration of 1%, and preserved at -50°C until it was put to use.

### (2) ELISA method

A dilution obtained by diluting the KTM-2001 antibody produced in Example 1 to a concentration of 10 µg/mL with the Tris-Hcl (pH 8.0) was dispensed to a 96-well microplate (manufactured by Nunc Corp) at a dose of 100 µL/well, and incubated at 4°C for 16 hours. After being deprived of the accompanying solution, 350 µL of the Tris-HCl (pH 8.0) containing 1%(w/v) BSA was added, and incubated at room temperature for two hours for blocking, and thereafter washed four times with the PBS (pH 7.4) containing 0.05% (v/v) Tween 20.

Next, fresh plasma of a patient of arteriosclerosis and that of a healthy person were respectively 1000-fold diluted with a sample diluting liquid (10 mmol/L phosphate buffer, containing 1% BSA, 5% polyether, and 140 mmol/L NaCl, pH 7.4), and the diluted plasmas were further diluted with the same sample diluting liquid to 4/5, 3/5, 2/5, and 1/5. The plasma thus diluted were respectively mixed with only the sample diluting liquid, which was dispensed to the wells in an amount of 100 µL/well, incubated at room temperature for two hours, and thereafter washed four times with the PBS (pH 7.4) containing the 0.05% (v/v) Tween 20.

Further, 100 µL of peroxidase-labeled anti-human Apo B antibody diluted in advance to 1,000 times with the PBS (pH 7.4) containing 1% (w/v) BSA was added to each of the wells and incubated at room temperature for 30 minutes. Next, the incubated mixture in the wells was washed four times with PBS (pH 7.4) containing 0.05% (v/v) Tween 20, and then color was developed with 100 µL of a coloring liquid (the TMBZ liquid, manufactured by TSI Corp) at 37°C for 30 minutes. After the reaction was terminated by the addition of 50 µL of 1 mol/L sulfuric acid, the absorbance at 450 nm was measured.

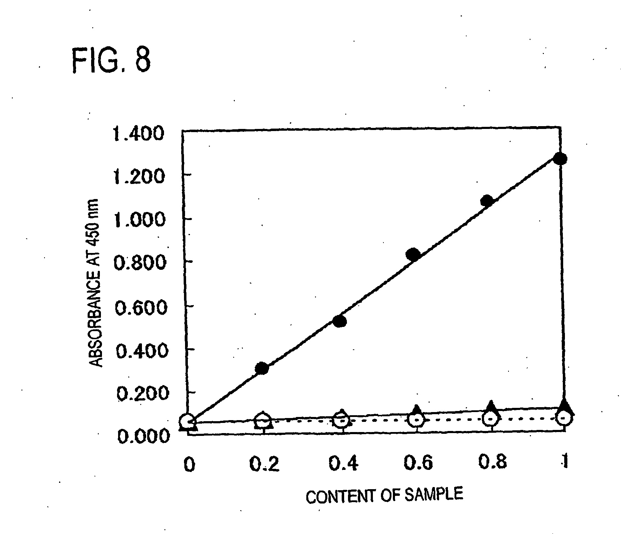

The results are shown in Fig. 8. It is clear from Fig. 8 that the plasma of the patient of arteriosclerosis contained a denatured lipoprotein having affinity for the KTM-2001 antibody and that the method of the present invention was capable of quantitatively determining this denatured lipoprotein.

### Example 5: Determination of denatured lipoprotein in vivo by use of KTM-285 antibody and correlation with disease

A dilution obtained by diluting the KTM-285 antibody produced in Example 2 to a concentration of 10 µg/mL with Tris-HCl (pH 8.0) was dispensed to a 96-well microplate (manufactured by Nunc Corp) in an amount of 100 µL/well and incubated at 4°C for 16 hours. After being deprived of the accompanying solution, 350 µL of Tris-HCl (pH 8.0) containing 1% (w/v) BSA was added thereto, and incubated at room temperature for two hours for blocking, and washed four times with the PBS (pH 7.4) containing 0.05% (v/v) Tween 20.

Next, a healthy person and a patient who was observed by coronarography to have a stenosis of not less than 75% in coronary artery were tested for denatured lipoprotein content in blood by the method of determination of the present invention. A serum was used as the biological sample. The subjects for the experiment were fasted for not less than 10 hours and, 10 mL of whole blood was collected from each of the subjects in the state of hunger in the early morning by the use of a vacuum blood collecting tube. After the blood samples were allowed to stand for coagulation at room temperature for 30 minutes, centrifuged at 3,000 rpm for 10 minutes to recover liquid components in a supernatant as a serum.

The serumwas diluted to 1, 000 times with a sample diluting liquid (10 mmol/L phosphate buffer, pH 7.4, containing 1% BSA, 5% polyether, and 140 mmo. /L of NaCl), dispensed to the wells in an amount of 100 µL/well, incubated at room temperature for two hours, and thereafter washed four times with the PBS (pH 7.4) containing 0.05% (v/v)of Tween 20.

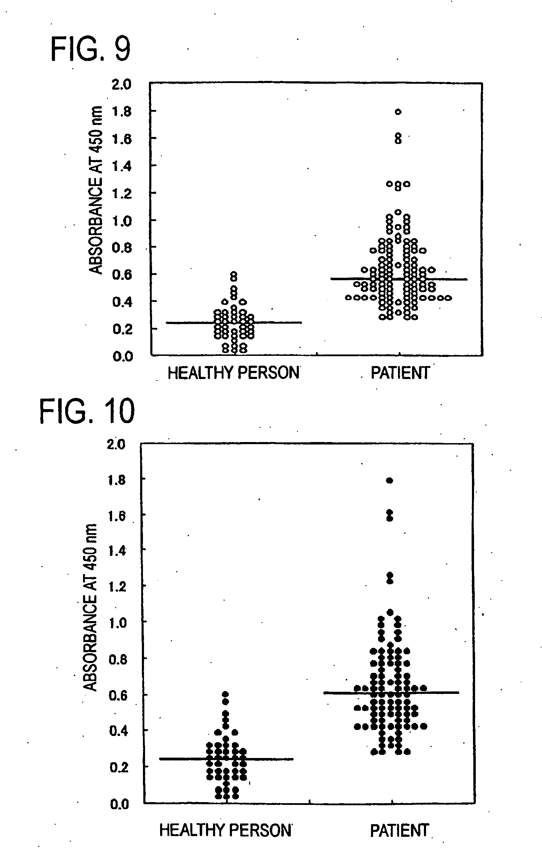

Further, 100 µL of peroxidase labeled anti-human Apo B antibody diluted in advance to 1,000 times the original volume with the PBS (pH 7.4) containing 1% (w/v) BSA was dispensed to the wells and incubated at room temperature for 30 minutes. Next, the incubated mixture in the wells was washed four times with PBS (pH 7.4) containing 0.05% (v/v) Tween 20, and color was developed with 100 µL of a coloring liquid (TMBZ liquid manufactured by TSI Corp) at 37°C for 30 minutes. After the reaction was terminated by the addition of 50 µL of 1 mol/L of sulfuric acid, the absorbance at 450 nm was measured. The results are shown in Fig. 9.

It is clear from Fig. 9 that the values obtained from the patient having not less than 75% of stenosis in coronary artery were significantly high as compared with those of the healthy person and that the disease of the coronary artery, cardiovascular disease, could be detected from the values obtained by determining a denatured lipoprotein by the present invention.

### Example 6: Determination of in vivo substance in patient of angina pectoris by the present invention

A healthy person and a patient of angina pectoris who showed a discernible fall in ST wave in exercise phonocardiogram were subjected to collection of 10 mL of whole blood by the use of a vacuum blood collecting tube. The blood samples were immediately centrifuged at 3,000 rpm for 10 minutes to recover liquid components in a supernatant, which was used as plasma. The recovered serums were subjected to determination by ELISA using the KTM-2001 antibody in the same manner as in Example 4. The results are shown in Fig. 10. It is clear from Fig. 10 that the values of the denatured lipoprotein in the patient of angina pectoris were significantly high as compared with those of the healthy person and that the disease of the circulatory organ could be detected by the values obtained by determining a denatured lipoprotein according to the present invention.

### Example 7: Determination of in vivo substance in patent of myocardial infarct by the present invention

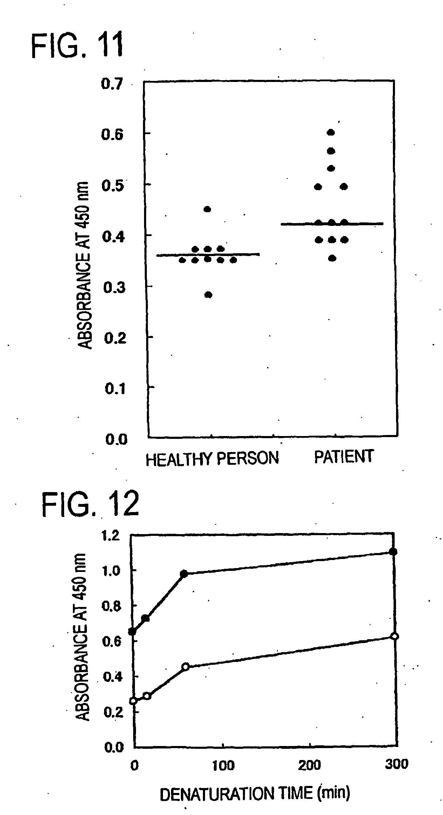

A patient of myocardial infarction who showed a symptom of pectoralgia, a confirmed rise of the ST wave in electrocardiogram, and a local abnormality of wall motion in echocardiogram and a healthy person were subjected to collection of 10 mL of the whole blood by means of a vacuum blood collection tube containing EDTA. The blood samples thus collected were immediately centrifuged at 3,000 rpm for 10 minutes to recover liquid components in a supernatant, which was used as plasma. To each of the plasmas were added an aqueous solution (10 mmol/L) of sodium ethylenediamine tetraacetate (EDTA-2Na) so as to give a final concentration of 1 mmol/L and the resultant mixtures were each adjusted to a density of 1.000 by the addition of NaBr. The mixtures thus obtained were each dispensed in centrifugal tubes, superposed sequentially with buffers adjusted to densities of 1.150, 1.063, 1.019, and 1.006 with NaBr, and centrifuged (120,000 x g) at 4°C for 24 hours. The contents in the tubes were each fractionated sequentially from the upper end downward and the fractions were tested for density with a refractometer. The fractions having densities of 1. 019 to 1. 063 were collected as LDL fractions. The LDL fractions thus obtained were immediately dialyzed against PBS containing 0.25 mmol/L EDTA. The recovered LDL fractions were examined by ELISA in the same manner as in Example 4. The results are shown in Fig. 11. It is clear from Fig. 11 that the values of denatured LDL from the patient of myocardial infarction were significantly high as compared with those of the healthy person and that the disease of the circulatory organ could be detected by the values obtained by determining a denatured LDL obtained by the present invention.

### Example 8: Determination of denatured LDL (1)

An healthy person and a patient who who was observed by coronarography to have a stenosis of not less than 75% in major coronary artery were fasted for not less than 10 hours and, subjected to collection of 10 mL of whole blood in the state of hunger in the early morning by the use of a vacuum blood collecting tube containing EDTA. The blood samples were immediately centrifuged at 3,000 rpm for 10 minutes and the liquid components in a supernatant were recovered to be used as plasma. The plasmas were each mixed with an aqueous solution (10 mmol/L) of sodium ethylenediamine tetraacetate (EDTA-2Na) so as to give a final concentration of 1 mmol/L and adjusted to a density of 1.000 by the addition of NaBr. The mixtures thus obtained were each dispensed in centrifugal tubes, superposed sequentially with buffers adjusted to densities of 1.150, 1.063, 1.019, and 1.006 with NaBr, and centrifuged (120,000 x g) at 4°C for 24 hours. The contents in the tubes were each fractionated sequentially from the upper end downward and the fractions were tested for density with a refractometer. The fractions having densities of 1.019 to 1.063 were collected as LDL fractions. The LDL fractions thus obtained were immediately dialyzed against PBS containing 0.25 mmol/L EDTA. The recovered purified LDL's were each divided into four parts in 15-mL conical tubes and stirred with vortex for a prescribed length of time to induce denaturation of lipoprotein. The denaturation of a given sample was confirmed by the fact that the absorbance at 680 nm increased. After the denaturation was completed, the purified LDL was examined by ELISA in the same manner as in Example 4. The results are shown in Fig. 12.

It is shown from Fig. 12 that the values obtained from the healthy person and the patient were both heightened in consequence of the denaturation caused by the stirring with vortex and that these values were further heightened by lengthening the duration of denaturation and exalting the degree of denaturation.

### Example 9: Determination of denaturated LDL (2)

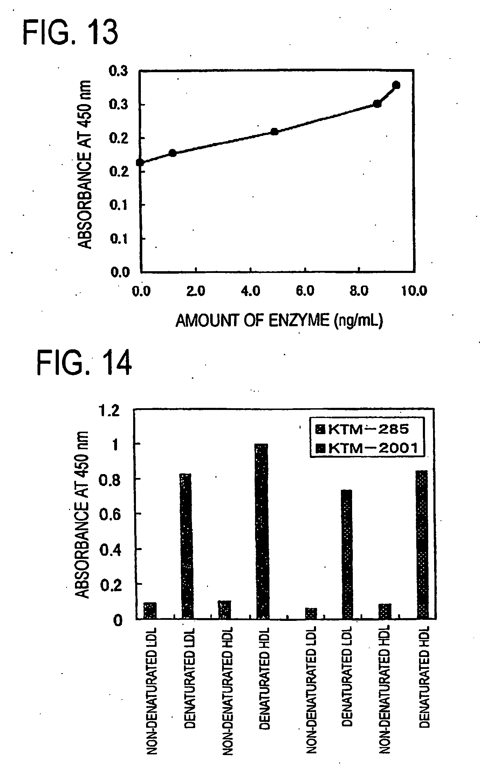

Healthy persons were fasted for not less than 10 hours and, subjected to collection of blood in the state of hunger in the early morning by a vacuum blood collecting tube containing EDTA. After the blood collection, the subjects were intravenously administered with heparin at a dose of 5 to 50 U/kg. After 15 minutes from the administration, 10 mL of the whole blood was ollected from each of the subjects with a vacuumblood collecting tube containing EDTA. The blood was immediately centrifuged at 3,000 rpm for 10 minutes. The liquid components in a supernatant was recovered to be used as plasma. Part of the plasma was stored as frozen at -50°C. To the recovered plasmas was added an aqueous solution (10 mmol/L) of sodium ethylenediamine tetraacetate (EDTA-2Na) so as to give a final concentration of 1 mmol/L and then adjusted to a density of 1.000 by the addition of NaBr. The resultant mixtures were each adjusted to a density of 1.000 by the addition of NaBr. The mixtures thus obtained were each dispensed in centrifugal tubes, superposed sequentially with buffers adjusted to densities of 1.150, 1.063, 1.019, and 1.006 with NaBr, and centrifuged (120,000 x g) at 4°C for 24 hours. The contents in the tubes were each fractionated sequentially from the upper end downward and the fractions were tested for density with a refractometer. The fractions having densities of 1.019 to 1.063 were collected as LDL fractions. The LDL fractions thus obtained were immediately dialyzed against PBS containing 0.25 mmol/L EDTA. The recovered LDL fractions were examined by ELISA in the same manner as in Example 4. The plasmas stored as frozen were each tested for lipoprotein lipase concentration with a reagent for the determination of lipoprotein lipase (manufactured by Daiichi Kagaku K.K.). The lipoprotein lipase concentration in plasma and the ELISA determination of the purified LDL's derived from plasma were compared and analyzed. The results are shown in Fig. 13.

It is shown from Fig. 13 that the values were heightened in proportion as the lipoprotein lipase concentrations of the purified LDL's derived from plasma increased and that the lipoprotein denatured with lipoprotein lipase could be determined by the method of determination of the present invention.

### Example 10: Determination of denatured LDL and denatured HDL (1) Preparation of HDL fraction in human plasma

The human plasma obtained from heparinized blood was mixed with EDTA so as to give a final concentration of 0.25 mmol/L. The resultant mixture was dispensed to supercentrifugal test tubes (4 mL in volume) each in an amount of 0.75 mL, superposed with 250 µL of 0.15 mol/L NaCl containing 0.3 mmol/L EDTA, and centrifuged at 185,000 x g at 10°C for 2.5 hours. While 150 pL of the upper layer in volume was discarded, 750 µL of the lower layer was mixed with 150 µL of a KBr solution (50 w/v%) to give a density of 1.063. The plasma having the density adjusted was transferred to the bottomof the test tube (4 mL in volume), centrifuged at 240, 000 x g at 10°C for 16 hours. The orange band (about 100 to 150 µL) in the upper layer was discarded and the remaining fraction was carefully recovered. The recovered fraction was added with KBr solution (50 w/v%) so as to give a density of 1.21, dispensed into the supercentrifugal test tubes, centrifuged at 244,000 x g for ten hours to recover 750 µL of an upper layer. The recovered upper layer was dialyzed against the PBS containing 0.25 mmol/L of EDTA at 4°C for six hours (three liters replaced three times at intervals of two hours). The HDL purity of HDL sample thus obtained was examined by showing a single band in agarose electrophoresis and lipid staining and then tested by Lowry method for the protein content with BSA as the standards. The value thus obtained was defined as the HDL concentration.

### (2) Preparation of denatured LDL and denatured HDL

The LDL purified in (1) of Example 2 and the HDL purified in (1) mentioned above were passed through a Sephadex G-25 column (1 cm x 30 cm, manufactured by Amersham-Parmacia Corp) equilibrated in advance with PBS, thereby removing EDTA contained therein. The resultant solution were subjected to determination of protein content by Lowry method using BSA as standard and then adjusted to 1 mg/mL with the PBS. To the solution thus obtained was added CuSO₄ so as to give a final concentration of 5 µmol/L and oxidized in the air at 37°C for three hours. After three hours, the oxidation was terminated by adding EDTA at a concentration of 0.01%. The oxidized product was immediately dialyzed against PBS containing 0.01% EDTA at 4°C. The dialyzate recovered was subj ected to determination of protein content by Lowry method with BSA as the standards. Thus, the concentrations of the denatured LDL and denatured HDL solutions were determined respectively.

### (3) Determination of denatured LDL and denatured HDL

The denatured LDL and the denatured HDL and the LDL and HDL solutions (10 µg/mL PBS) prior to oxidation which were produced as described above, each 50 µL in volume, were dispensed a 96-well microtiter plate (manufactured by Nunc Corp) and allowed to stand at 4°C overnight. After the plate was washed three times with PBS, 250 µL of 1% BSA/PBS solution was dispensed to the wells and allowed to stand at room temperature for one hour. The wells were washed three times with PBS to prepare a plate for the reaction. The KTM-285 antibody obtained in Example 2 and the KTM-2001 antibody obtained in Example 1, which were respectively diluted with 0.1 mol/L phosphate buffer (pH 7.4) containing 0.1% BSA at a concentration of 100 ng/mL, were dispensed each in 50 µL to the reaction plate, mixed together, and allowed to stand 4°C overnight. After the reaction, the plate was washed five times with PBS containing 0.05% Tween 20, mixed with 50 µL of POD-labeled anti-mouse immunoglobulins rabbit IgG, and allowed to react at room temperature for one hour. After the reaction, the plate was washed five times with PBS containing 0.05% Tween 20, color was developed with 50 µL of a TMB coloring solution (manufactured by Intergene Corp), and allowed to react at room temperature for 30 minutes. Finally, 50 µL of a reaction terminating liquid was added, the absorbance at 450 nm was deteremined with a microplate reader. The reults are shown in Fig. 14. It is clear fromTable 14 that the KTM-285 antibody and the KTM-2001 antibody did not react with LDL and HDL prior to denaturation but reacted with denatured LDL and denatured HDL. The result indicates that the denatured LDL and the denatured HDL could be determined.

### Example 11: Determination of denatured lipoprotein

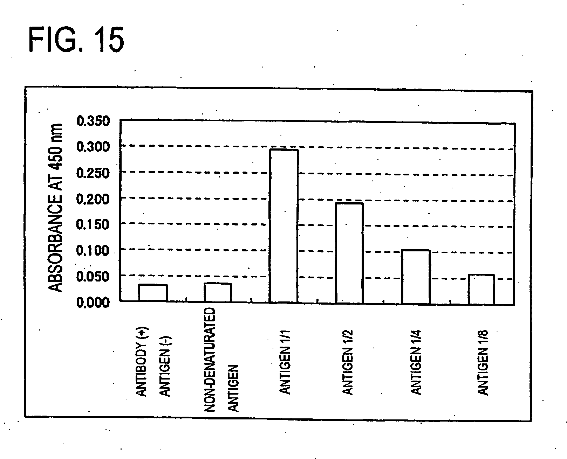

The denatured LDL prepared in Example 10 in a 96-well microtiter plate (manufactured by Nunc Corp) was mixed with 250 µL of 1% BSA/PBS solution, allowed to stand at room temperature for one hour. Then, the wells of the plate were washed three times with PBS to prepare a plate for the reaction. The denatured LDL produced in Example 10, the LDL solution (170 µg/mL PBS) prior to denaturation, and the denatured LDL diluted to 1/2, 1/4, and 1/8 were each dispensed to the wells of the plate in an amount of 100 µL/well, and the KTM-285 antibody obtained in Example 2 diluted with 0.1% BSA-containing 0.1 mol/L phosphate buffer (pH 7.4) at a concentration of 970 µg/mL was dispensed thereto in an amount of 50 µL. They were mixed and then allowed to stand at 24°C for 20 minutes. After the reaction, the absorbance at 450 nm was determined with a microplate reader. The results are shown in Fig. 15. It is clear from Fig. 15 that the KTM-285 antibody did not react with LDL prior to denaturation but reacted with denatured LDL which had undergone the denaturation and that the denatured LDL could be determined by the method of the present invention. These results were reproduced by using the denatured HDL produced in Example 10.

### Industrial Applicability

According to the present invention, a method for determining a denatured lipoprotein in a biological sample which comprises determining the denatured lipoprotein in the biological sample by the use of an antibody against phosphocholine, a method for determining a denatured lipoprotein in a biological sample which comprises determining the denatured lipoprotein in the biological sample by using an antibody against phosphocholine and an antibody against a lipoprotein and a method for detecting cardiovascular disease which comprises determining a denatured lipoprotein in the biological sample by using an antibody against phosphocholine and an antibody against a lipoprotein and detecting the circulatory disease from the value obtained by the determination are provided. Also described herein is a reagent for determining a denatured lipoprotein containing an antibody against phosphocholine, and a reagent for determining a denatured lipoprotein containing an antibody against phosphocholine and an antibody against a lipoprotein.

### "Free Text of Sequence Listing"

SEQ. ID. No. 3 - Explanation of artificial sequence: Synthesis DNA
SEQ. ID. No. 4 - Explanation of artificial sequence: Synthesis DNA
SEQ. ID. No. 5 - Explanation of artificial sequence: Synthesis DNA
SEQ. ID. No. 6 - Explanation of artificial sequence: Synthesis DNA
SEQ. ID. No. 7 - Explanation of artificial sequence: Synthesis DNA
SEQ. ID. No. 8 - Explanation of artificial sequence: Synthesis DNA
SEQ. ID. No. 9 - Explanation of artificial sequence: Synthesis DNA
SEQ. ID. No. 10 - Explanation of artificial sequence: Synthesis DNA

## Claims

1. A method for the quantitative determination of a denatured lipoprotein in a biological sample, which comprises contacting the biological sample with an antibody against phosphocholine and determining a formed immune complex.

2. A method as claimed in claim 1, further comprising contacting the biological sample with an antibody against lipoprotein.

3. A method as claimed in claim 2, wherein the denatured lipoprotein is a denatured low-density lipoprotein and the antibody against lipoprotein is an antibody against a low-density lipoprotein.

4. A method as claimed in claim 2, wherein the denatured lipoprotein is a denatured high-density lipoprotein and the antibody against lipoprotein is an antibody against a high-density lipoprotein.

5. A method as claimed in claim 1 or claim 2, which is for the quantitative determination of a denatured high-density lipoprotein and a denatured low-density lipoprotein, wherein the biological sample is contacted with an antibody against a high-density lipoprotein and an antibody against a low-density lipoprotein.

6. A method according to claim 4 or claim 5, wherein the antibody against the high-density lipoprotein is an antibody against an Apo A protein.

7. A method according to claim 3 or claim 5, wherein the antibody against the low-density lipoprotein is an antibody against an Apo B protein.

8. A method for the quantitative determination of a denatured lipoprotein (a) in a biological sample, which comprises contacting the biological sample with an antibody against phosphocholine and an antibody against an apoprotein (a) and determining a formed immune complex.

9. A method for the detection of cardiovascular disease, wherein a method as claimed in any preceding claim is used.

## Patentansprüche

1. Verfahren zur quantitativen Bestimmung eines denaturierten Lipoproteins in einer biologischen Probe, umfassend das in Kontakt bringen der biologischen Probe mit einem Antikörper gegen Phosphocholin und das Bestimmen eines gebildeten Immunkomplexes.

2. Verfahren nach Anspruch 1, weiterhin umfassend das in Kontakt bringen der biologischen Probe mit einem Antikörper gegen Lipoprotein.

3. Verfahren nach Anspruch 2, worin das denaturierte Lipoprotein ein denaturiertes Lipoprotein geringer Dichte ist und der Antikörper gegen Lipoprotein ein Antikörper gegen ein Lipoprotein geringer Dichte ist.

4. Verfahren nach Anspruch 2, worin das denaturierte Lipoprotein ein denaturiertes Lipoprotein hoher Dichte ist und der Antikörper gegen Lipoprotein ein Antikörper gegen ein Lipoprotein hoher Dichte ist.

5. Verfahren nach Anspruch 1 oder 2, das zur quantitativen Bestimmung eines denaturierten Lipoproteins hoher Dichte und eines denaturierten Lipoproteins geringer Dichte dient, worin die biologischen Probe mit einem Antikörper gegen ein Lipoprotein hoher Dichte und einem Antikörper gegen ein Lipoprotein geringer Dichte in Kontakt gebracht wird.

6. Verfahren nach Anspruch 4 oder 5, worin der Antikörper gegen das Lipoprotein hoher Dichte ein Antikörper gegen ein Apo A-Protein ist.

7. Verfahren nach Anspruch 3 oder 5, worin der Antikörper gegen das Lipoprotein geringer Dichte ein Antikörper gegen ein Apo B-Protein ist.

8. Verfahren zur quantitativen Bestimmung eines denaturierten Lipoproteins (a) in einer biologischen Probe, umfassend das in Kontakt bringen der biologischen Probe mit einem Antikörper gegen Phosphocholin und einem Antikörper gegen ein Apoprotein (a) und das Bestimmen eines gebildeten Immunkomplexes.

9. Verfahren zum Nachweis einer kardiovaskulären Erkrankung, worin ein Verfahren nach einem der vorhergehenden Ansprüche verwendet wird.

## Revendications

1. Procédé pour la détermination quantitative d'une lipoprotéine dénaturée dans un échantillon biologique, qui comprend la mise en contact de l'échantillon biologique avec un anticorps dirigé contre de la phosphocholine et la détermination d'un complexe immun formé.

2. Procédé tel que revendiqué dans la revendication 1, comprenant de plus la mise en contact de l'échantillon biologique avec un anticorps dirigé contre une lipoprotéine.

3. Procédé tel que revendiqué dans la revendication 2, dans lequel la lipoprotéine dénaturée est une lipoprotéine de basse densité dénaturée et l'anticorps dirigé contre une lipoprotéine est un anticorps dirigé contre une lipoprotéine de basse densité.

4. Procédé tel que revendiqué dans la revendication 2, dans lequel la lipoprotéine dénaturée est une lipoprotéine de haute densité dénaturée et l'anticorps dirigé contre une lipoprotéine est un anticorps dirigé contre une lipoprotéine de haute densité.

5. Procédé tel que revendiqué dans la revendication 1 ou la revendication 2, qui est destiné à la détermination quantitative d'une lipoprotéine de haute densité dénaturée et d'une lipoprotéine de basse densité dénaturée, dans lequel l'échantillon biologique est mis en contact avec un anticorps dirigé contre une lipoprotéine de haute densité, et un anticorps dirigé contre une lipoprotéine de basse densité.

6. Procédé selon la revendication 4 ou la revendication 5, dans lequel l'anticorps dirigé contre la lipoprotéine de haute densité est un anticorps dirigé contre une protéine Apo A.

7. Procédé selon la revendication 3 ou la revendication 5, dans lequel l'anticorps dirigé contre la lipoprotéine de basse densité est un anticorps dirigé contre une protéine Apo B.

8. Procédé pour la détermination quantitative d'une lipoprotéine dénaturée (a) dans un échantillon biologique, qui comprend la mise en contact de l'échantillon biologique avec un anticorps dirigé contre de la phosphocholine et un anticorps dirigé contre une apoprotéine (a) et la détermination d'un complexe immun formé.

9. Procédé pour la détection d'une maladie cardio-vasculaire, dans lequel un procédé tel que revendiqué dans l'une quelconque des revendications précédentes est utilisé.
